# EUROPEAN PATENT SPECIFICATION

(11) **EP 1 377 567 B1**
(45) Date of publication and mention of the grant of the patent: **21.12.2005**
(21) Application number: 02735247.5
(22) Date of filing: 11.04.2002
(51) Int. Cl.: C07D 401/06, C07D 409/14, C07D 215/16, A61K 31/47, A61K 31/4709, A61P 11/00, A61P 25/00, C07D 417/06, C07D 487/04

(54) **QUINOLINE-4-CARBOXAMIDE DERIVATIVES AS NK-3 AND NK-2 RECEPTOR ANTAGONISTS**
CHINOLIN-4-CARBOXAMIDDERIVATE ALS NK-3 UND NK-2 REZEPTOR ANTAGONISTEN
DERIVES DE QUINOLINE-4-CARBOXAMIDE COMME ANTAGONISTES DE RECEPTEURS DE NK-3 ET NK-4

(30) Priority: 11.04.2001 GB 0109123; 11.03.2002 GB 0205649
(43) Date of publication of application: 07.01.2004
(73) Proprietor: GlaxoSmithKline S.p.A., 37135 Verona (IT)
(72) Inventor: FARINA, Carlo, Nikem Research S.r.l., I-20021 Baranzate di Bollate (IT); GAGLIARDI, Stefania, NiKem Research srl, I-20021 Baranzante di Bollate (IT); GIARDINA, Giuseppe A. M., Nikem Research S.r.l., I-20021 Baranzate di Bollate (IT); MARTINELLI, Marisa, NiKem Research S.r.l., I-20021 Baranzante di Bollate (IT)
(74) Representative: Rutter, Keith
(86) International application number: PCT/EP2002/004066
(87) International publication number: WO 2002/083663

(56) References cited:
- WO-A-00/31037
- WO-A-00/31038
- WO-A-97/19926
- WO-A-98/52942
- GIARDINA G A M ET AL: "DISCOVERY OF A NOVEL CLASS OF SELECTIVE NON-PEPTIDE ANTAGONISTS FOR THE HUMAN NEUROKININ-3 RECEPTOR. 2. IDENTIFICATION OF (S)-N- (1-PHENYLPROPYL)-3-HYDROXY-2-PHENYLQUINOLI NE-4-CARBOXAMIDE (SB 223412)" JOURNAL OF MEDICINAL CHEMISTRY, AMERICAN CHEMICAL SOCIETY. WASHINGTON, US, vol. 42, no. 6, 1999, pages 1053-1065, XP000882756 ISSN: 0022-2623
- SWAIN C J: "PATENT UPDATE CENTRAL & PERIPHERAL NERVOUS SYSTEMS NEUROKININ RECEPTOR ANTAGONISTS" EXPERT OPINION ON THERAPEUTIC PATENTS, ASHLEY PUBLICATIONS, GB, vol. 6, no. 4, 1996, pages 367-378, XP000956432 ISSN: 1354-3776
- GIARDINA G A M ET AL: "2-PHENYL-4-QUINOLINECARBOXYAMIDES: A NOVEL CLASS OF POTENT AND SELECTIVE NON-PEPTIDE COMPETITIVE ANTAGONISTS FOR THE HUMAN NEUROKININ-3 RECEPTOR" JOURNAL OF MEDICINAL CHEMISTRY, AMERICAN CHEMICAL SOCIETY. WASHINGTON, US, vol. 39, no. 12, 7 June 1996 (1996-06-07), pages 2281-2284, XP000197077 ISSN: 0022-2623

## Description

The present invention relates to novel compounds, in particular to novel quinoline derivatives, to processes for the preparation of such compounds, to pharmaceutical compositions containing such compounds and to the use of such compounds in medicine.

The mammalian peptide Neurokinin B (NKB) belongs to the Tachykinin (TK) peptide family which also include Substance P (SP) and Neurokinin A (NKA). Pharmacological and molecular biological evidence has shown the existence of three subtypes of TK receptor (NK₁, NK₂ and NK₃) and NKB binds preferentially to the NK₃ receptor although it also recognises the other two receptors with lower affinity (Maggi et al, **1993**, *J. Auton. Pharmacol*., *13,* 23-93).

Selective peptidic NK₃ receptor antagonists are known (Drapeau, **1990** *Regul. Pept.,* 31, 125-135), and findings with peptidic NK₃ receptor agonists suggest that NKB, by activating the NK₃ receptor, has a key role in the modulation of neural input in airways, skin, spinal cord and nigro-striatal pathways (Myers and Undem, 1993, *J.Physiol.,* 470, 665-679; Counture et al., 1993, *Regul. Peptides,* 46, 426-429; Mccarson and Krause, 1994, *J. Neurosci.,* 14 (2), 712-720; Arenas et al. 1991, *J.Neurosci.,* 11, 2332-8). However, the peptide-like nature of the known antagonists makes them likely to be too labile from a metabolic point of view to serve as practical therapeutic agents.

International Patent Application, Publication Number WO 00/58307 describes a series of aryl fused 2,4-disubstituted pyridines, such as naphthyridine derivatives, which are stated to exhibit biological activity as NK₃ receptor antagonists.

The compounds of the present invention are quinoline derivatives. Other quinoline derivatives have been described previously as selective NK₃ antagonists. For example, International Patent Application, Publication Numbers, WO 95/32948 and WO 96/02509 describe a series of selective and potent NK₃ receptor antagonists.

International Patent Application, Publication Number WO 00/64877 describes a series of 2-aminoquinolinecarboxamides as neurokinin receptor ligands.

International Patent Application, Publication Number, WO 00/58303 describes a series of 4-substituted quinoline derivatives which are stated to be NK₃ and/or GABA(A) receptor ligands. Such compounds are characterised by the presence of a nitrogen-containing heterocyclic moiety at the C(4) position of the quinoline ring.

International Patent Application, Publication Numbers, WO 97/21680, WO 98/52942, WO 00/31037 and WO 00/31038 describe compounds which have biological activity as combined NK₃ and NK₂ receptor antagonists.

Copending International Patent Application Numbers, PCT/EP01/13833, PCT/EP01/14140 and PCT/EP01/13832 also describe compounds that have biological activity as combined NK₃ and NK₂ receptor antagonists.

We have now discovered a further novel class of non-peptide NK₃ antagonists which are far more stable from a metabolic point of view than the known peptidic NK₃ receptor antagonists and are of potential therapeutic utility. These compounds also have NK₂ antagonist activity and are therefore considered to be of potential use in the prevention and treatment of a wide variety of clinical conditions, which are characterised by overstimulation of the Tachykinin receptors, in particular NK₃ and NK₂.

These conditions include respiratory diseases, such as chronic obstructive pulmonary disease (COPD), asthma, airway hyper-reactivity, cough; inflammatory diseases such as inflammatory bowel disease, psoriasis, fibrositis, osteoarthritis, rheumatoid arthritis and inflammatory pain; neurogenic inflammation or peripheral neuropathy, allergies such as eczema and rhinitis; ophthalmic diseases such as ocular inflammation, conjunctivitis, vernal conjuctivitis and the like; cutaneous diseases, skin disorders and itch, such as cutaneous wheal and flare, contact dermatitis, atopic dermatitis, urticaria and other eczematoid dermatitis; adverse immunological reactions such as rejection of transplanted tissues and disorders related to immune enhancement or suppression such as systhemic lupus erythematosis; gastrointestinal (GI) disorders and diseases of the GI tract such as disorders associated with the neuronal control of viscera such as ulcerative colitis, Crohn's disease, irritable bowel syndrome (IBS), gastro-exophageous reflex disease (GERD); urinary incontinence and disorders of the bladder function; renal disorders; increased blood pressure, proteinuria, coagulopathy and peripheral and cerebral oedema following pre-eclampsia in pregnancies (hereinafter referred to as the 'Primary Conditions').

Certain of these compounds also show CNS activity and hence are considered to be of particular use in the treatment of disorders of the central nervous system such as anxiety, depression, psychosis and schizophrenia; neurodegenerative disorders such as AIDS related dementia, senile dementia of the Alzheimer type, Alzheimer's disease, Down's syndrome, Huntingdon's disease, Parkinson's disease, movement disorders and convulsive disorders (for example epilepsy); demyelinating diseases such as multiple sclerosis and amyotrophic lateral sclerosis and other neuropathological disorders such as diabetic neuropathy, AIDS related neuropathy, chemotherapy-induced neuropathy and neuralgia; addiction disorders such as alcoholism; stress related somatic disorders; reflex sympathetic dystrophy such as shoulder/hand syndrome; dysthymic disorders; eating disorders (such as food intake disease); fibrosing and collagen diseases such as scleroderma and eosinophilic fascioliasis; disorders of the blood flow caused by vasodilatation and vasospastic diseases such as angina, migraine and Reynaud's disease and pain or nociception, for example, that is attributable to or associated with any of the foregoing conditions especially the transmission of pain in migraine, (hereinafter referred to as the 'Secondary Conditions').

The compounds of formula (I) are also considered to be useful as diagnostic tools for assessing the degree to which neurokinin-3 and neurokinin-2 receptor activity (normal, overactivity or underactivity) is implicated in a patient's symptoms.

Certain compounds of the present invention have also been found to exhibit surprisingly advantageous pharmacochemical properties.

According to the present invention, there is provided a compound of formula (I) below or a pharmaceutically acceptable salt or solvate thereof: 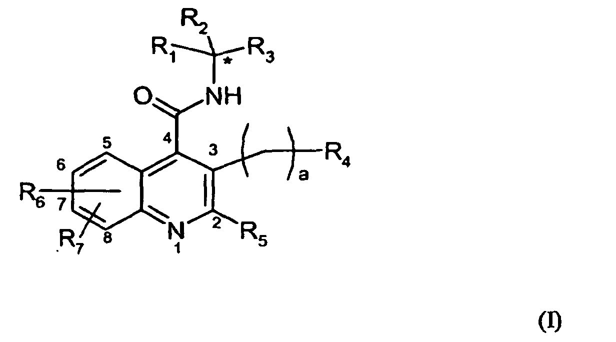 wherein, R₁, R₂, R₃, R₄, R₅, R₆ and R7 are as defined in Table 1.

Particularly preferred compounds of formula (I) which are of special interest as agents useful in the treatment and/or prophylaxis of conditions which are characterised by overstimulation of the Tachykinin receptors, in particular NK₃ and NK₂, are those listed in Table 4 below.

The compounds of formula (I) may have at least one asymmetric centre - for example the carbon atom labelled with an asterisk (*) in the compound of formula (I) - and therefore may exist in more than one stereoisomeric form. The invention extends to all such stereoisomeric forms and to mixtures thereof, including racemates. In particular, the invention includes compounds wherein the asterisked carbon atom in formula (I) has the stereochemistry shown in formula (Ia): 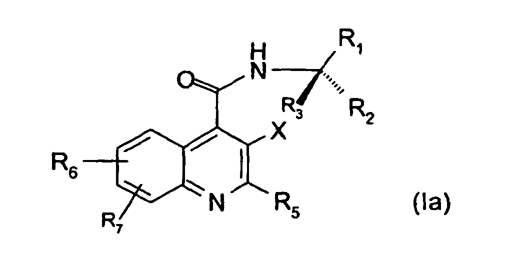 wherein R₁, R₂, R₃, R₅, R₆, and R₇ are as defined in relation to formula (I), and X represents the moiety

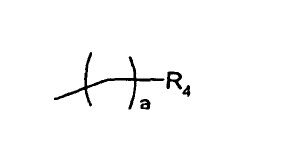

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels.

A substantially pure form will generally contain at least 50% (excluding normal pharmaceutical additives), preferably 75%, more preferably 90% and still more preferably 95% of the compound of formula (I) or its salt or solvate.

One preferred pharmaceutically acceptable form is the crystalline form, including such form in pharmaceutical composition. In the case of salts and solvates the additional ionic and solvent moieties must also be non-toxic.

Suitable salts are pharmaceutically acceptable salts.

Suitable pharmaceutically acceptable salts include the acid addition salts with the conventional pharmaceutical acids, for example maleic, hydrochloric, hydrobromic, phosphoric, acetic, fumaric, salicylic, citric, lactic, mandelic, tartaric, succinic, benzoic, ascorbic and methanesulphonic.

Suitable pharmaceutically acceptable salts include salts of acidic moieties of the compounds of formula (I) when they are present, for example salts of carboxy groups or phenolic hydroxy groups.

Suitable salts of acidic moieties include metal salts, such as for example aluminium, alkali metal salts such as lithium, sodium or potassium, alkaline earth metal salts such as calcium or magnesium and ammonium or substituted ammonium salts, for example those with lower alkylamines such as triethylamine, hydroxy alkylamines such as 2-hydroxyethylamine, bis-(2-hydroxyethyl)-amine or tri-(2-hydroxyethyl)-amine, cycloalkylamines such as bicyclohexylamine, or with procaine, dibenzylpiperidine, N-benzyl-β-phenethylamine, dehydroabietylamine, N,N'-bisdehydroabietylamine, glucamine, N-methylglucamine or bases of the pyridine type such as pyridine, collidine, quinine or quinoline.

Suitable solvates are pharmaceutically acceptable solvates.

Suitable pharmaceutically acceptable solvates include hydrates.

The invention also provides in one aspect a process for the preparation of a compound of formula (I), or a salt thereof and/or a solvate thereof, which process comprises reacting a compound of formula (II) or an active derivative thereof: 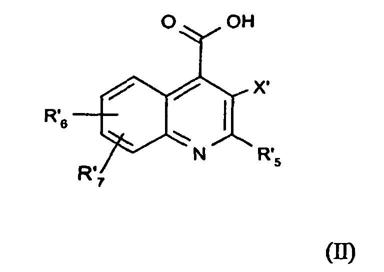 wherein R'₆, R'₇, R'₅ and X' are R₆, R₇, R₅ and X respectively as hereinbefore defined in relation to formula (I) or (Ia), or a group convertible to R₆, R₇, R₅ and X respectively; with a compound of formula (III): 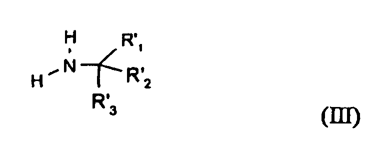 wherein R'₁, R'₂, and R'₃ are R₁, R₂, and R₃ as defined for formula (I) or a group or atom convertible to R₁, R₂, and R₃ respectively; to form a compound of formula (Ib): 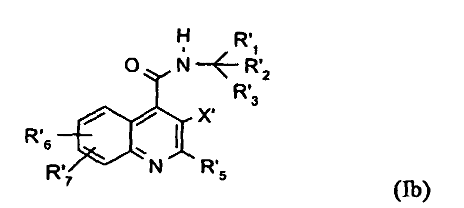 wherein R'₁, R'₂, R'₃, X', R'₅, R'₆ and R'₇ are as defined above, and thereafter carrying out one or more of the following optional steps:
(i) converting any one of R'₁, R'₂, R'₃, X', R'₅, R'₆ and R'₇ to R₁, R₂, R₃, X, R₅, R₆ and R₇ respectively as required, to obtain a compound of formula (I);
(ii) converting a compound of formula (I) into another compound of formula (I); and
(iii) preparing a salt of the compound of formula (I) and/or a solvate thereof.

Suitable groups convertible into other groups include protected forms of said groups.

Suitably R'₁, R'₂, R'₃, X', R'₅, R'₆ and R'₇ each represents R₁, R₂, R₃, X, R₅, R₆ and R₇ respectively or a protected form thereof.

It is favoured if the compound of formula (II) is present as an active derivative.

A suitable active derivative of a compound of formula (II) is a transient activated form of the compound of formula (II) or a derivative wherein the carboxy group of the compound of formula (II) has been replaced by a different group or atom, for example by an acyl halide, preferably a chloride, or an acylazide or a carboxylic acid anhydride.

Other suitable active derivatives include: a mixed anhydride formed between the carboxyl moiety of the compound of formula (II) and an alkyl chloroformate; an activated ester, such as a cyanomethyl ester, thiophenyl ester, p-nitrophenyl ester, p-nitrothiophenyl ester, 2,4,6-trichlorophenyl ester, pentachlorophenyl ester, pentafluorophenyl ester, N-hydroxy-phtalimido ester, N-hydroxypiperidine ester, N-hydroxysuccinimide ester, N-hydroxy benzotriazole ester; alternatively, the carboxy group of the compound of formula (II) may be activated using a carbodiimide or N,N'-carbonyldiimidazole.

The reaction between the compound of formula (II) or the active derivative thereof and the compound of formula (III) is carried out under the appropriate conventional conditions for the particular compounds chosen. Generally, when the compound of formula (II) is present as an active derivative the reaction is carried out using the same solvent and conditions as used to prepare the active derivative, preferably the active derivative is prepared *in situ* prior to forming the compound of formula (Ib) and thereafter the compound of formula (I) or a salt thereof and/or a solvate thereof is prepared.

For example, the reaction between an active derivative of the compound of formula (II) and the compound of formula (III) may be carried out:
(a) by first preparing an acid chloride and then coupling said chloride with the compound of formula (III) in the presence of an inorganic or organic base in a suitable aprotic solvent such as dimethylformamide (DMF) at a temperature in a range from -70 to 50°C (preferably in a range from -10 to 20°C); or
(b) by treating the compound of formula (II) with a compound of formula (III) in the presence of a suitable condensing agent, such as for example N,N'-carbonyl diimidazole (CDI) or a carbodiimide such as dicyclohexylcarbodiimide (DCC) or N-dimethylaminopropyl-N'-ethylcarbodiimide, preferably in the presence of N-hydroxybenzotriazole (HOBT) to maximise yields and avoid racemization processes (see *Synthesis,* 453, 1972), or O-benzotriazol-1-yl-N,N,N',N'-tetramethyluroniumhexafluorophosphate (HBTU), in an aprotic solvent, such as a mixture of acetonitrile (MeCN) and tetrahydrofuran (THF), for example a mixture in a volume ratio of from 1:9 to 7:3 (MeCN:THF), at any temperature providing a suitable rate of formation of the required product, such as a temperature in the range of from -70 to 50°C, preferably in a range of from -10 to 25°C, for example at 0°C.

A preferred reaction is set out in Scheme 1 shown below: 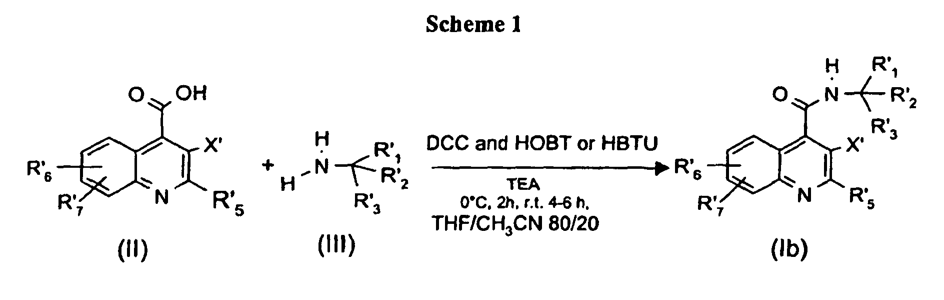 wherein R'₁, R'₂, R'₃, X', R'₅, R'₆ and R'₇ are as defined above.

In the case in which the corresponding alkyl (such as methyl or ethyl) ester of compound (II) is utilised, an hydrolysis to compound (II) is required before conversion to compound (Ib) in Scheme 1. Such hydrolysis can be carried out under acidic conditions, such 10-36% hydrochloric acid at a temperature in the range between 30 and 100 °C.

It will be appreciated that a compound of formula (Ib) may be converted to a compound of formula (I), or one compound of formula (I) may be converted to another compound of formula (I) by interconversion of suitable substituents. Thus, certain compounds of formula (I) and (Ib) are useful intermediates in forming other compounds of the present invention.

Accordingly, in a further aspect the invention provides a process for preparing a compound of formula (I), or a salt thereof and/or a solvate thereof, which process comprises converting a compound of the above defined formula (Ib) wherein at least one of R'₁, R'₂, R'₃, X', R'₅, R'₆ and R'₇ is not R₁, R₂, R₃, X, R₅, R₆ or R₇ respectively, thereby to provide a compound of formula (I); and thereafter, as required, carrying out one or more of the following optional steps:
(i) converting a compound of formula (I) into another compound of formula (I); and
(ii) preparing a salt of the compound of formula (I) and/or a solvate thereof.

Suitably, in the compound of formula (Ib) the variables R'₁, R'₂, R'₃, X', R'₅, R'₆ and R'₇ are R₁, R₂, R₃, X, R₅, R₆ and R₇ respectively or they are protected forms thereof.

The above mentioned conversions, protections and deprotections are carried out using the appropriate conventional reagents and conditions and are further discussed below.

A chiral compound of formula (III) wherein R₂ is a C₅ or C₇ cycloalkyl group, R₃ is methyl and R₁ is H are described in J. Org. Chem. (1996), 61 (12), 4130-4135. A chiral compound of formula (III) wherein R₂ is phenyl, R₃ is isopropyl and R₁ is H is a known compound described in for example Tetrahedron Lett. (1994), 35(22), 3745-6.

The compounds of formula (III) are known commercially available compounds or they can be prepared from known compounds by known methods, or methods analogous to those used to prepare known compounds, for example the methods described in Liebigs Ann. der Chemie, (1936), 523, 199.

In some embodiments of the invention, a compound of formula (II) or the corresponding alkyl (such as methyl or ethyl) ester is prepared by reacting a compound of formula (IV) or the corresponding alkyl (such as methyl or ethyl) ester: 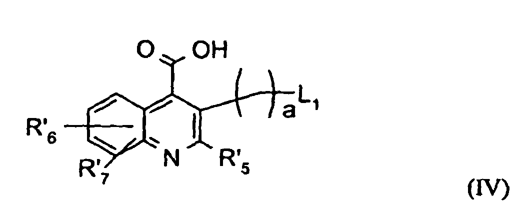 wherein R'₆, R'₇, R'₅ and a are as defined above and L₁ represents a halogen atom such as a bromine atom, with a compound of formula (V):

H―R'₄ (V)

wherein R'₄ is R₄ as defined in relation to formula (I) or a protected form thereof.

Suitably, R'₄ is R₄.

Suitably, reaction between the compounds of formulae (IV) or the corresponding alkyl (such as methyl or ethyl) ester and (V) is carried out under conventional amination conditions, for example when L₁ is a bromine atom then the reaction is conveniently carried out in an aprotic solvent, such as tetrahydrofuran or dimethylformamide at any temperature providing a suitable rate of formation of the required product, usually at ambient temperature; preferably the reaction is carried out in the presence of triethylamine (TEA) or K₂CO₃.

The compounds of formula (V) are known, commercially available compounds or they can be prepared using methods analogous to those used to prepare known compounds; for example the methods described in the Chemistry of the Amino Group, Patais (Ed.), Interscience, New York 1968; Advanced Organic Chemistry, March J, John Wiley & Sons, New York, 1992 ; J. Heterocyclic Chem. (1990), 27, 1559; Synthesis (1975), 135, Bioorg. Med. Chem. Lett. (1997), 7, 555, or Protective Groups in Organic Synthesis (second edition), Wiley Interscience, (1991) or other methods mentioned herein.

In cases where a is 1, a compound of formula (IV) or the corresponding alkyl (such as methyl or ethyl) ester may be prepared by appropriate halogenation of a compound of formula (VI) or the corresponding alkyl (such as methyl or ethyl) ester: 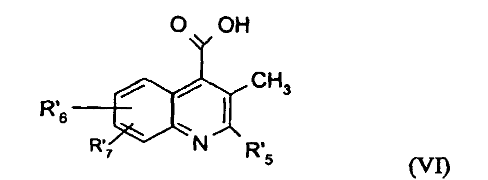 wherein R'₆, R'₇ and R'₅ are as defined above in relation to formula (II).

Suitable halogenation reagents are conventional reagents depending upon the nature of the halogen atom required, for example when L₁is bromine a preferred halogenation reagent is N-bromosuccinimide (NBS).

The halogenation of the compound of formula (VI) or the corresponding alkyl (such as methyl or ethyl) ester is suitably carried out under conventional conditions, for example bromination is carried out by treatment with NBS in an inert solvent, such as carbon tetrachloride CCl₄, or 1,2-dichloroethane or CH₃CN, at any temperature providing a suitable rate of formation of the required product, suitably at an elevated temperature such as a temperature in the range of 60°C to 100°C, for example 80°C; preferably the reaction is carried out in the presence of a catalytic amount of benzoyl peroxide.

A compound of formula (VI) is conveniently prepared by reacting a compound of formula (VII): 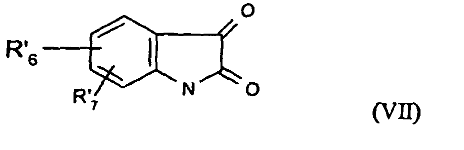 wherein R'₆ and R'₇ are as defined in relation to formula (II), with a compound of formula (X)III:

R₅'― CO―CH₂―Me (XIII)

wherein R'₅ is as defined in relation to formula (II).

The reaction between the compounds of formula (VII) and (XIII) is conveniently carried out using Pfitzinger reaction conditions (see for example J. Prakt. Chem. 33, 100 (1886), J. Prakt. Chem. 38, 582 (1888), J. Chem. Soc. 106 (1948) and Chem. Rev. 35, 152 (1944)). For example in an alkanolic solvent such as ethanol, at any temperature providing a suitable rate of formation of the required product, but generally at an elevated temperature, such as the reflux temperature of the solvent, and preferably in the presence of a base such as potassium hydroxide or potassium tert-butoxide. It will be appreciated that the Pfitzinger reaction can be also carried out in presence of an acid, such as acetic acid or hydrochloric acid, at any temperature providing a suitable rate of formation of the required product, but generally at an elevated temperature, as described in .J. Med. Chem. 38, 906 (1995).

The compounds of formula (VII) are known compounds or they are prepared according to methods used to prepare known compounds for example those disclosed in J. Org. Chem. 21, 171 (1955); J. Org. Chem. 21, 169 (1955).

Alternatively a compound of formula (VI) may be conveniently prepared by reacting a compound of formula (XIV) 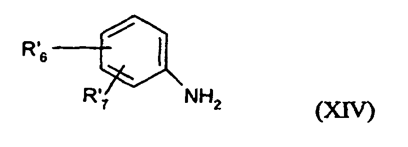 wherein R'₆ and R'₇ are as defined in relation to formula (II), with a compound of formula (XV):

R₅'―CHO (XV)

wherein R'₅ is as defined in relation to formula (II) in presence of oxobutyric acid.

The reaction between the compounds of formula (XIV) and (XV) is conveniently carried out using Doebner reaction conditions (see for example Chem. Ber. 29, 352 (1894); Chem. Revs. 35, 153, (1944); J. Chem. Soc. B, 1969, 805), for example in an alcoholic solvent such as ethanol, at any temperature providing a suitable rate of formation of the required product, but generally at an elevated temperature, such as the reflux temperature of the solvent.

The compounds of formula (XIV) and (XV) are known compounds or they are prepared according to methods used to prepare known compounds for example as described in *Vogel's Textbook of Practical Organic Chemistry.*

In some alternative embodiments of the invention, a compound of formula (II) wherein X' represents 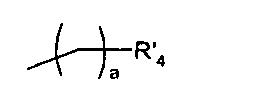 is prepared by reacting a compound of formula (VII) as defined above with a compound of formula (VIII):

R₅'―CO―CH₂ ―(CH₂)a―T₅ (VIII)

wherein R'₅ is as defined in relation to formula (II), and T₅ is a group

―R'₄―Y

where Y is a protecting group such as a benzyl group, particularly a protecting group which is stable in basic conditions such as a terbutoxycarbonyl group; and a is as defined in relation to formula (II); and thereafter as required removing any protecting group, for example by dehydrogenation, and/or converting any group T₅ to R₄.

The reaction between the compounds of formula (VII) and (VIII) is conveniently carried out using Pfitzinger reaction conditions (see for example J. Prakt. Chem. 33, 100 (1886), J. Prakt. Chem. 38, 582 (1888), J. Chem. Soc. 106 (1948) and Chem. Rev. 35, 152 (1944)), for example in an alkanolic solvent such as ethanol, at any temperature providing a suitable rate of formation of the required product, but generally at an elevated temperature, such as the reflux temperature of the solvent, and preferably in the presence of a base such as potassium hydroxide or potassium tert-butoxide.

Protected forms of R₄ will vary according to the particular nature of the group being protected but will be chosen in accordance with normal chemical practice.

Groups convertible to R₄ include groups dictated by conventional chemical practice to be required and to be appropriate, depending upon the specific nature of the R₄ under consideration.
Suitable deprotection methods for deprotecting protected forms of R₄ and conversion methods for converting T₅ to R₄ will be those used conventionally in the art depending upon the particular groups under consideration with reference to standard texts such as Greene, T.W. and Wuts, P.G.M. Protective Groups in Organic Synthesis, John Wiley & Sons Inc. New York, 1991 (Second Edt.) or in Kocienski, P.J. Protecting groups. George Thieme Verlag, New York, 1994 and Chemistry of the Amino Group, Patais (Ed.), Interscience, New York 1968; or Advanced Organic Chemistry, March J, John Wiley & Sons, New York, 1992.

A compound of formula (VIII) is prepared from a compound of formula (IX):

R₅'―CO―CH₂―(CH₂)ₐ―OH (IX)

wherein R'₅ is as defined in relation to formula (II) and a is as defined in relation to formula (VIII), by first halogenating, preferably brominating, or mesylating the compound of formula (IX) and thereafter reacting the halogenation or mesylation product so formed with a compound capable of forming a group T₅ so as to provide the required compound of formula (VII).

When T₅ is a group R₄, a compound capable of forming a group T₅ is a compound of the above defined formula (V).

The halogenation of the compound of formula (IX) is suitably carried out using a conventional halogenation reagent. Mesylation is conveniently carried out using mesyl chloride in an inert solvent such as methylene dichloride, at a temperature below room temperature, such as 0°C, preferably in the presence of triethylamine.

The reaction conditions between the compound of formula (IX) and the compound capable of forming a group T₅ will be those conventional conditions dictated by the specific nature of the reactants, for example when the T₅ required is a group R₄ and the required compound capable of forming a group T₅ is a compound of the above defined formula (V), then the reaction between the halogenation or mesylation product of the compound of formula (IX) and the compound of formula (V) is carried out under analogous conditions to those described for the reaction between the compounds of formulae (IV) and (V).

Other compounds capable of forming a group T₅ will depend upon the particular nature of T₅, but will be those appropriate compounds dictated by conventional chemical practice with reference to standard texts such as Chemistry of the Amino Group, Patais (Ed.), Interscience, New York 1968; and Advanced Organic Chemistry, March J, John Wiley & Sons, New York, 1992.

A compound of formula (IX) may be prepared by reacting a compound of formula (X): 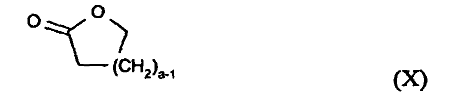 wherein a is as defined in relation to formula (VIII), with a lithium salt of formula (XI):

R'₅Li (XI)

wherein R'₅ is as defined in relation to formula (II).

The reaction between the compounds of formulae (X) and (XI) can be carried out in an aprotic solvent, such as diethyl-ether at any temperature providing a suitable rate of formation of the required product, usually at a low temperature such as in the range of-10°C to -30°C, for example -20°C.

The compounds of formula (VII) are known compounds or they are prepared according to methods used to prepare known compounds for example those disclosed in J. Org. Chem. 21, 171 (1955); J. Org. Chem. 21, 169 (1955).

The compounds of formula (X) and (XI) are known compounds or they are prepared according to methods used to prepare known compounds for example those disclosed by Krow G. R. in Organic Reactions, Vol 43, page 251, John Wiley & Sons Inc. 1994 (for the compounds of formula (X)) and Organometallics in Synthesis, Schlosser M.(Ed), John Wiley & Sons Inc. 1994 (for the compounds of formula (XI)).

In another aspect, the present invention provides a process for the preparation of a compound of formula (I), or a salt thereof and/or a solvate thereof, wherein a is 1, which process comprises reacting a compound of formula (XVI): 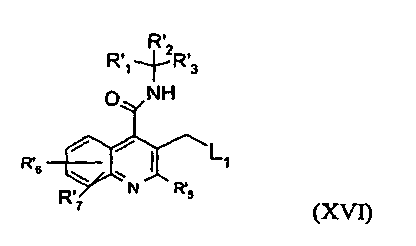 wherein each of R'₁, R'₂, R'₃, R'₅, R'₆, and R'₇ is respectively R₁, R₂, R₃, R₅, R₆, or R₇ as defined above or a group convertible to R₁, R₂, R₃, R₅, R₆, or R₇ respectively as defined above providing R'₂ is not aromatic in character, and L₁ represents a halogen atom such as a bromine atom, with a compound of formula (XVII):

H―R'₄ (XVII)

wherein R'₄ is R₄ as defined in relation to formula (I) or a protected form thereof or a group convertible thereto; and thereafter carrying out one or more of the following optional steps:
(i) converting any one of R'₁, R'₂, R'₃, R'₄, R'₅, R'₆ and R'₇ to R₁, R₂, R₃, R₄, R₅, R₆ and R₇ respectively as required, to obtain a compound of formula (I);
(ii) converting a compound of formula (I) into another compound of formula (I); and
(iii) preparing a salt of the compound of formula (I) and/or a solvate thereof.

Protected forms of R₄ will vary according to the particular nature of the group being protected but will be chosen in accordance with normal chemical practice.

Groups convertible to R₄ include groups dictated by conventional chemical practice to be required and to be appropriate, depending upon the specific nature of the R₄ under consideration.

Suitable deprotection methods for deprotecting protected forms of R₄ and conversion methods for converting R'₄ to R₄ will be those used conventionally in the art depending upon the particular groups under consideration with reference to standard texts such as Greene, T.W. and Wuts, P.G.M. Protective Groups in Organic Synthesis, John Wiley & Sons Inc. New York, 1991 (Second Edt.) or in Kocienski, P.J. Protecting groups. George Thieme Verlag, New York, 1994 and Chemistry of the Amino Group, Patais (Ed.), Interscience, New York 1968; or Advanced Organic Chemistry, March J, John Wiley & Sons, New York, 1992.

Suitable groups convertible into other groups include protected forms of said groups.

Advantageously, a compound of formula (XVII) will be a compound of formula (V) as defined above.

Suitably R'₁, R'₂, R'₃, R'₄, R'₅, R'₆ and R'₇ each represents R₁, R₂, R₃, R₄, R₅, R₆ and R₇ respectively or a protected form thereof.

Suitable deprotection methods for deprotecting protected forms of R₁, R₂, R₃, R₄, R₅, R₆ and R₇ and conversion methods for converting R'₁, R'₂, R'₃, R'₄, R'₅, R'₆ and R'₇ to R'₁, R₂, R₃, R₄, R₅, R₆ and R₇ respectively will be those used conventionally in the art depending upon the particular groups under consideration with reference to standard texts such as Greene, T.W. and Wuts, P.G.M. Protective Groups in Organic Synthesis, John Wiley & Sons Inc. New York, 1991 (Second Edt.) or in Kocienski, P.J. Protecting groups. George Thieme Verlag, New York, 1994 and Chemistry of the Amino Group, Patais (Ed.), Interscience, New York 1968; or Advanced Organic Chemistry, March J, John Wiley & Sons, New York, 1992.

Suitably, reaction between the compounds of formulae (XVI) and (XVII) is carried out under conventional amination conditions, for example when L₁ is a bromine atom then the reaction is conveniently carried out in an aprotic solvent, such as tetrahydrofuran or dimethylformamide or acetonitrile at any temperature providing a suitable rate of formation of the required product, usually at ambient temperature; preferably the reaction is carried out in the presence of triethylamine (TEA), sodium hydride or K₂CO₃.

The compounds of formula (XVII) are known, commercially available compounds or they can be prepared using methods analogous to those used to prepare known compounds; for example the methods described in the Chemistry of the Amino Group, Patais (Ed.), Interscience, New York 1968; Advanced Organic Chemistry, March J, John Wiley & Sons, New York, 1992 ; J. Heterocyclic Chem. (1990), 27, 1559; Synthesis (1975), 135, Bioorg. Med. Chem. Lett. (1997), 7, 555, or Protective Groups in Organic Synthesis (second edition), Wiley Interscience, (1991) or other methods mentioned herein.

A compound of formula (XVI) is prepared by appropriate halogenation of a compound of formula (XVIII): 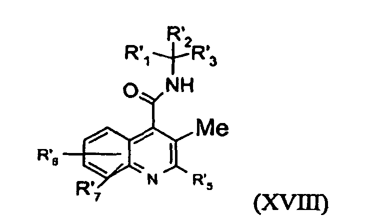 wherein R'₁, R'₂, R'₃, R'₅, R'₆, and R'₇ are as defined above in relation to formula (XVI).

Suitable halogenation reagents are conventional reagents depending upon the nature of the halogen atom required, for example when L₁ is bromine a preferred halogenation reagent is N-bromosuccinimide (NBS).

The halogenation of the compound of formula (XVIII) is carried out under conventional conditions, for example bromination is carried out by treatment with NBS in an inert solvent, such as carbon tetrachloride CCl₄, or 1,2-dichloroethane or CH₃CN, at any temperature providing a suitable rate of formation of the required product, suitably at an elevated temperature such as a temperature in the range of 60°C to 100°C, for example 80°C; preferably the reaction is carried out in the presence of a catalytic amount of benzoyl peroxide.

Suitably, the compound of formula (XVIII) may be prepared by reacting a compound of formula (VI) as defined above or an active derivative thereof with a compound of formula (III) as defined above wherein R'₂ is not aromatic in character.

It is favoured if the compound of formula (VI) is present in the reaction mix as an active derivative, as hereinbefore described.

The reaction between the compound of formula (VI) or the active derivative thereof and the compound of formula (III) is carried out under the appropriate conventional conditions for the particular compounds chosen. Generally, when the compound of formula (VI) is present as an active derivative the reaction is carried out using the same solvent and conditions as used to prepare the active derivative, preferably the active derivative is prepared *in situ* prior to forming the compound of formula (XVIII).

For example, the reaction between an active derivative of the compound of formula (VI) and the compound of formula (III) may be carried out:
(a) by first preparing an acid chloride and then coupling said chloride with the compound of formula (III) in the presence of an inorganic or organic base in a suitable aprotic solvent such as methylene dichloride or tetrahydrofuran at a temperature in a range from -70 to 50°C (preferably in a range from 20°C to reflux temperature); or
(b) by treating the compound of formula (VI) with a compound of formula (III) in the presence of a suitable condensing agent, such as for example N,N'-carbonyl diimidazole (CDI) or a carbodiimide such as dicyclohexylcarbodiimide (DCC) or N-dimethylaminopropyl-N'-ethylcarbodiimide, preferably in the presence of N-hydroxybenzotriazole (HOBT) to maximise yields and avoid racemization processes (see *Synthesis,* 453, 1972), or O-benzotriazol-1-yl-N,N,N',N'-tetramethyluroniumhexafluorophosphate (HBTU), in an aprotic solvent, such as a mixture of acetonitrile (MeCN) and tetrahydrofuran (THF), for example a mixture in a volume ratio of from 1:9 to 7:3 (MeCN:THF), at any temperature providing a suitable rate of formation of the required product, such as a temperature in the range of from -70 to 50°C, preferably in a range of from -10 to 25°C, for example at 0°C.

A preferred reaction is set out in Scheme 2 shown below:

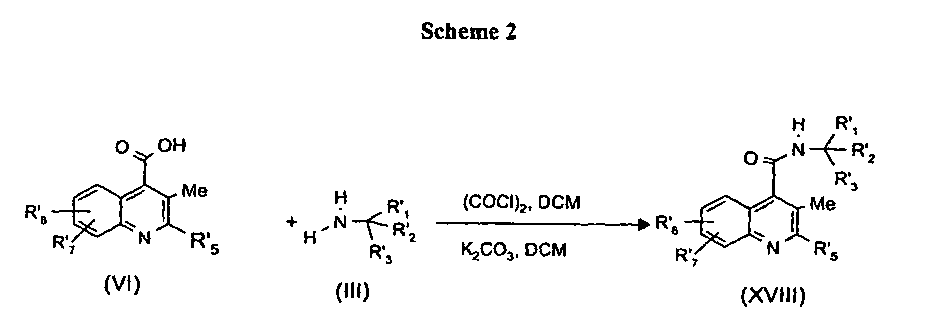

In the case in which the corresponding alkyl (such as methyl or ethyl) ester of compounds (VI) is utilised, a hydrolysis is required before conversion to compound (XVIII) in Scheme 2. Such hydrolysis can be carried out under acidic conditions, such 10-36% hydrochloric acid at a temperature in the range between 30 and 100 °C.

As hereinbefore mentioned, the compounds of formula (I) may exist in more than one stereoisomeric form - and the process of the invention may produce racemates as well as enantiomerically pure forms. Accordingly, a pure enantiomer of a compound of formula (I) can be obtained by reacting a compound of the above defined formula (II) with an appropriate enantiomerically pure primary amine of formula (IIIa) or (IIIc): 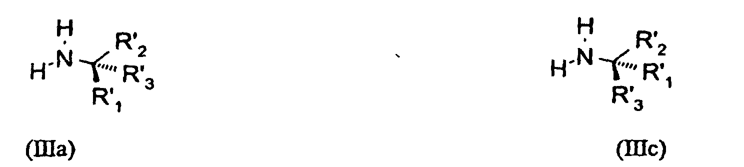 wherein R'₁, R'₂ and R'₃ are as defined above, to obtain a compound of formula (I'a) or (I'c): 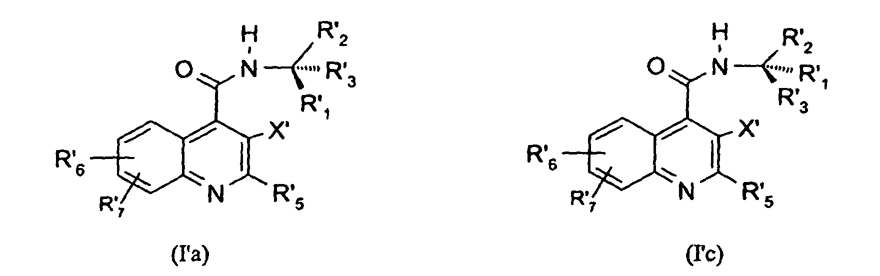 wherein R'₁, R'₂, R'₃, X', R'₅, R'₆, and R'₇ are as defined above.

Compounds of formula (I'a) or (I'c) may subsequently be converted to compounds of formula (Ia) or (Ic) by the methods of conversion mentioned before: 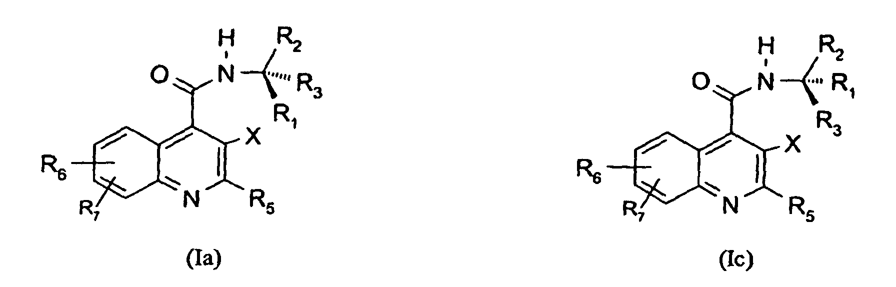 wherein R₁, R₂, R₃, X, R₅, R₆, and R₇ are as defined above.

Suitably, in the above mentioned compounds of formulae (Ia), (Ic), (I'a), (I'c), (IIIa) and (IIIc) R₁ represents hydrogen.

An alternative method for separating optical isomers is to use conventional, fractional separation methods in particular fractional crystallization methods. Thus, a pure enantiomer of a compound of formula (I) is obtained by fractional crystallisation of a diastereomeric salt formed by reaction of the racemic compound of formula (I) with an optically active strong acid resolving agent, such as camphosulphonic acid, tartaric acid, O,O'-di-p-toluoyltartaric acid or mandelic acid, in an appropriate alcoholic solvent, such as ethanol or methanol, or in a ketonic solvent, such as acetone. The salt formation process should be conducted at a temperature between 20°C and 80°C, preferably at 50°C.

A suitable conversion of one compound of formula (I) into a further compound of formula (I) involves converting one group X into another group X by for example:
(i) converting a ketal into a ketone, by such as mild acidic hydrolysis, using for example dilute hydrochloric acid;
(ii) reducing a ketone to a hydroxy group by use of a borohydride reducing agent;
(iii) converting a carboxylic ester group into a carboxyl group using basic hydrolysis;
   and/or
(iv) reducing a carboxylic ester group to a hydroxymethyl group, by use of a borohydride reducing agent.

As indicated above, where necessary, the conversion of any group R'₁, R'₂, R'₃, X', R'₅, R'₆, and R'₇ into R₁, R₂, R₃, X, R₅, R₆, and R₇ which as stated above are usually protected forms of R₁, R₂, R₃, X, R₅, R₆, or R₇ may be carried out using appropriate conventional conditions such as the appropriate deprotection procedure.

It will be appreciated that in any of the above mentioned reactions any reactive group in the substrate molecule may be protected and deprotected according to conventional chemical practice, for example as described by Greene, T.W. and Wuts, P.G.M. Protective Groups in Organic Synthesis, John Wiley & Sons Inc. New York, 1991 (Second Edt.) or in Kocienski, P.J. Protecting groups. George Thieme Verlag, New York, 1994.

Suitable protecting groups in any of the above mentioned reactions are those used conventionally in the art. Thus, for example suitable hydroxy protecting groups include benzyl or trialkylsilyl groups.

The methods of formation and removal of such protecting groups are those conventional methods appropriate to the molecule being protected. Thus for example a benzyloxy group may be prepared by treatment of the appropriate compound with a benzyl halide, such as benzyl bromide, and thereafter, if required, the benzyl group may be conveniently removed using catalytic hydrogenation or a mild ether cleavage reagent such as trimethylsilyl iodide or boron tribromide.

As indicated above, the compounds of formula (I) have useful pharmaceutical properties.

Accordingly the present invention also provides a compound of formula (I), or a pharmaceutically acceptable salt or solvate thereof, for use as an active therapeutic substance.

In particular, the present invention also provides a compound of formula (I), or a pharmaceutically acceptable salt or solvate thereof, for the treatment or prophylaxis of the Primary and Secondary Conditions.

The present invention further provides a pharmaceutical composition comprising a compound of formula (I), or a pharmaceutically acceptable salt or solvate thereof, and a pharmaceutically acceptable carrier.

The present invention also provides the use of a compound of formula (I), or a pharmaceutically acceptable salt or solvate thereof, in the manufacture of a medicament for the treatment of the Primary and Secondary Conditions.

As mentioned above the Primary conditions include respiratory diseases, such as chronic obstructive pulmonary disease (COPD), asthma, airway hyperreactivity, cough; inflammatory diseases such as inflammatory bowel disease, psoriasis, fibrositis, osteoarthritis, rheumatoid arthritis and inflammatory pain; neurogenic inflammation or peripheral neuropathy, allergies such as eczema and rhinitis; ophthalmic diseases such as ocular inflammation, conjunctivitis, vernal conjuctivitis and the like; cutaneous diseases, skin disorders and itch, such as cutaneous wheal and flare, contact dermatitis, atopic dermatitis, urticaria and other eczematoid dermatitis; adverse immunological reactions such as rejection of transplanted tissues and disorders related to immune enhancement or suppression such as systemic lupus erythematosis; gastrointestinal (GI) disorders and diseases of the GI tract such as disorders associated with the neuronal control of viscera such as ulcerative colitis, Crohn's disease, irritable bowel syndrome (IBS), gastro-exophageous reflex disease (GERD); urinary incontinence and disorders of the bladder function; renal disorders; increased blood pressure, proteinuria, coagulopathy and peripheral and cerebral oedema following pre-eclampsia in pregnancies.

As mentioned above, the Secondary conditions include disorders of the central nervous system such as anxiety, depression, psychosis and schizophrenia; neurodegenerative disorders such as AIDS related dementia, senile dementia of the Alzheimer type, Alzheimer's disease, Down's syndrome, Huntingdon's disease, Parkinson's disease, movement disorders and convulsive disorders (for example epilepsy); demyelinating diseases such as multiple sclerosis and amyotrophic lateral sclerosis and other neuropathological disorders such as diabetic neuropathy, AIDS related neuropathy, chemotherapy-induced neuropathy and neuralgia; addiction disorders such as alcoholism; stress related somatic disorders; reflex sympathetic dystrophy such as shoulder/hand syndrome; dysthymic disorders; eating disorders (such as food intake disease); fibrosing and collagen diseases such as scleroderma and eosinophilic fascioliasis; disorders of the blood flow caused by vasodilatation and vasospastic diseases such as angina, migraine and Reynaud's disease and pain or nociception, for example, that is attributable to or associated with any of the foregoing conditions especially the transmission of pain in migraine.

Such a medicament, and a composition of this invention, may be prepared by admixture of a compound of the invention with an appropriate carrier. It may contain a diluent, binder, filler, disintegrant, flavouring agent, colouring agent, lubricant or preservative in conventional manner.

These conventional excipients may be employed for example as in the preparation of compositions of known agents for treating the conditions.

Preferably, a pharmaceutical composition of the invention is in unit dosage form and in a form adapted for use in the medical or veterinarial fields. For example, such preparations may be in a pack form accompanied by written or printed instructions for use as an agent in the treatment of the conditions.

The suitable dosage range for the compounds of the invention depends on the compound to be employed and on the condition of the patient. It will also depend, inter alia, upon the relation of potency to absorbability and the frequency and route of administration.

The compound or composition of the invention may be formulated for administration by any route, and is preferably in unit dosage form or in a form that a human patient may administer to himself in a single dosage. Advantageously, the composition is suitable for oral, rectal, topical, parenteral, intravenous or intramuscular administration. Preparations may be designed to give slow release of the active ingredient.

Compositions may, for example, be in the form of tablets, capsules, sachets, vials, powders, granules, lozenges, reconstitutable powders, or liquid preparations, for example solutions or suspensions, or suppositories.

The compositions, for example those suitable for oral administration, may contain conventional excipients such as binding agents, for example syrup, acacia, gelatine, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinyl-pyrrolidone, sodium starch glycollate or microcrystalline cellulose; or pharmaceutically acceptable setting agents such as sodium lauryl sulphate.

Solid compositions may be obtained by conventional methods of blending, filling, tabletting or the like. Repeated blending operations may be used to distribute the active agent throughout those compositions employing large quantities of fillers. When the composition is in the form of a tablet, powder, or lozenge, any carrier suitable for formulating solid pharmaceutical compositions may be used, examples being magnesium stearate, starch, glucose, lactose, sucrose, rice flour and chalk. Tablets may be coated according to methods well known in normal pharmaceutical practice, in particular with an enteric coating. The composition may also be in the form of an ingestible capsule, for example of gelatine containing the compound, if desired with a carrier or other excipients.

Compositions for oral administration as liquids may be in the form of, for example, emulsions, syrups, or elixirs, or may be presented as a dry product for reconstitution with water or other suitable vehicle before use. Such liquid compositions may contain conventional additives such as suspending agents, for example sorbitol, syrup, methyl cellulose, gelatine, hydroxyethylcellulose, carboxymethylcellulose, aluminium stearate gel, hydrogenated edible fats; emulsifying agents, for example lecithin, sorbitan monooleate, or acacia; aqueous or non-aqueous vehicles, which include edible oils, for example almond oil, fractionated coconut oil, oily esters, for example esters of glycerine, or propylene glycol, or ethyl alcohol, glycerine, water or normal saline; preservatives, for example methyl or propyl p-hydroxybenzoate or sorbic acid; and if desired conventional flavouring or colouring agents.

The compounds of this invention may also be administered by a non-oral route. In accordance with routine pharmaceutical procedure, the compositions may be formulated, for example for rectal administration as a suppository. They may also be formulated for presentation in an injectable form in an aqueous or non-aqueous solution, suspension or emulsion in a pharmaceutically acceptable liquid, e.g. sterile pyrogen-free water or a parenterally acceptable oil or a mixture of liquids. The liquid may contain bacteriostatic agents, anti-oxidants or other preservatives, buffers or solutes to render the solution isotonic with the blood, thickening agents, suspending agents or other pharmaceutically acceptable additives. Such forms will be presented in unit dose form such as ampoules or disposable injection devices or in multi- dose forms such as a bottle from which the appropriate dose may be withdrawn or a solid form or concentrate which can be used to prepare an injectable formulation.

The compounds of this invention may also be administered by inhalation, via the nasal or oral routes. Such administration can be carried out with a spray formulation comprising a compound of the invention and a suitable carrier, optionally suspended in, for example, a hydrocarbon propellant.

Preferred spray formulations comprise micronised compound particles in combination with a surfactant, solvent or a dispersing agent to prevent the sedimentation of suspended particles. Preferably, the compound particle size is from about 2 to 10 microns.

A further mode of administration of the compounds of the invention comprises transdermal delivery utilising a skin-patch formulation. A preferred formulation comprises a compound of the invention dispersed in a pressure sensitive adhesive which adheres to the skin, thereby permitting the compound to diffuse from the adhesive through the skin for delivery to the patient. For a constant rate of percutaneous absorption, pressure sensitive adhesives known in the art such as natural rubber or silicone can be used.

As mentioned above, the effective dose of compound depends on the particular compound employed, the condition of the patient and on the frequency and route of administration. A unit dose will generally contain from 20 to 1000 mg and preferably will contain from 30 to 500 mg, in particular 50, 100, 150, 200, 250, 300, 350, 400, 450, or 500 mg. The composition may be administered once or more times a day for example 2, 3 or 4 times daily, and the total daily dose for a 70 kg adult will normally be in the range 100 to 3000 mg. Alternatively the unit dose will contain from 2 to 20 mg of active ingredient and be administered in multiples, if desired, to give the preceding daily dose.

No unacceptable toxicological effects are expected with compounds of the invention when administered in accordance with the invention.

The present invention also provides a method for the treatment and/or prophylaxis of the Primary and Secondary Conditions in mammals, particularly humans, which comprises administering to the mammal in need of such treatment and/or prophylaxis an effective, non-toxic pharmaceutically acceptable amount of a compound of formula (I) or a pharmaceutically acceptable salt or solvate thereof.

The activity of the compounds of the present invention, as NK₃ ligands, is determined by their ability to inhibit the binding of the radiolabelled NK₃ ligands, [¹²⁵I]-[Me-Phe⁷]-NKB or [³H]-Senktide, to guinea-pig and human NK₃ receptors (Renzetti et al, **1991,** *Neuropeptide, 18,* 104-114; Buell et al, **1992,** *FEBS, 299(1),* 90-95; Chung et al, **1994,** *Biochem. Biophys. Res. Commun., 198(3),* 967-972).

The binding assays utilised allow the determination of the concentration of the individual compound required to reduce by 50% the [¹²⁵I]-[Me-Phe⁷]-NKB and [³H]-Senktide specific binding to NK₃ receptor in equilibrium conditions (IC50).

Binding assays provide for each compound tested a mean IC₅₀ value of 2-5 separate experiments performed in duplicate or triplicate. The most potent compounds of the present invention show IC₅₀ values in the range 0.1-1000 nM. The NK₃-antagonist activity of the compounds of the present invention is determined by their ability to inhibit senktide-induced contraction of the guinea-pig ileum (Maggi et al, **1990,** *Br. J. Pharmacol., 101,* 996-1000) and rabbit isolated iris sphincter muscle (Hall et al., **1991,** *Eur. J. Pharmacol., 199,* 9-14) and human NK₃ receptors-mediated Ca⁺⁺ mobilisation (Mochizuki et al, **1994,** *J*. *Biol. Chem., 269,* 9651-9658). Guinea-pig and rabbit *in-vitro* functional assays provide for each compound tested a mean K_{B} value of 3-8 separate experiments, where K_{B} is the concentration of the individual compound required to produce a 2-fold rightward shift in the concentration-response curve of senktide. Human receptor functional assay allows the determination of the concentration of the individual compound required to reduce by 50% (IC₅₀ values) the Ca⁺⁺ mobilisation induced by the agonist NKB. In this assay, the compounds of the present invention behave as antagonists.

The activity of the compounds of the present invention, as NK-2 ligands, is determined by their ability to inhibit the binding of the radiolabelled NK-2 ligands, [¹²⁵I]-NKA or [³H]-NKA, to human NK-2 receptors (Aharony et al, **1992**, *Neuropeptide, 23*, 121-130).

The binding assays utilised allow the determination of the concentration of the individual compound required to reduce by 50% the [¹²⁵I]-NKA and [³H]-NKA specific binding to NK2 receptor in equilibrium conditions (IC₅₀).

Binding assays provide for each compound tested a mean IC₅₀ value of 2-5 separate experiments performed in duplicate or triplicate. The most potent compounds of the present invention show IC₅₀ values in the range 0.5-1000 nM, such as 1-1000 nM. The NK-2-antagonist activity of the compounds of the present invention is determined by their ability to inhibit human NK-2 receptor-mediated Ca⁺⁺ mobilisation (Mochizuki et al, **1994**, *J*. *Biol. Chem., 269*, 9651-9658). Human receptor functional assay allows the determination of the concentration of the individual compound required to reduce by 50% (IC₅₀ values) the Ca⁺⁺ mobilisation induced by the agonist NKA. In this assay, the compounds of the present invention behave as antagonists.

The therapeutic potential of the compounds of the present invention in treating the conditions can be assessed using rodent disease models.

As stated above, the compounds of formula (I) are also considered to be useful as diagnostic tools. Accordingly, the invention includes a compound of formula (I) for use as diagnostic tools for assessing the degree to which neurokinin-3 and neurokinin-2 receptor activity (normal, overactivity or underactivity) is implicated in a patient's symptoms. Such use comprises the use of a compound of formula (I) as an antagonist of said activity, for example including but not restricted to Tachykinin agonist-induced inositol phosphate turnover or electrophysiological activation, of a cell sample obtained from a patient. Comparison of such activity in the presence or absence of a compound of formula (I), will disclose the degree of NK-3 and NK-2 receptor involvement in the mediation of agonist effects in that tissue.

The following Descriptions illustrate the preparation of the intermediates, whereas the following Examples illustrate the preparation of the compounds of the invention.

### Descriptions and Examples

### Description 1. 3-Methyl-2-phenylquinoline 4-carboxylic acid

Isatine (40 g, 0.272 mol) is suspended in EtOH (11) and KOH (62.8 g, 1.1 mol). Suspension is stirred for 30 min. Propiophenone (36.2 cc, 0.272 mol) was added and the reaction was refluxed for 4 h. The reaction was left overnight at room temperature and then EtOH was evaporated under vacuum. The solid was dissolved in water (400 ml) and washed with Et₂O. The aqueous phase was acidified with citric acid (saturated solution) and a solid was obtained. The solid was filtered by suction, washed with water and dried in oven to obtain the title compound, mp > 280°C.

### Description 2. ((S)-1-Cyclohexyl-ethyl)-3-methyl-2-phenylquinoline-4-carboxamide

4-Carboxy-3-methyl-2-phenylquinoline (40 g, 0.152 mol) prepared as in Description 1, was suspended in CH₂Cl₂ (600 ml) and oxalyl chloride (6.6 ml, 0.311 mol) was added dropwise at 0° C under magnetic stirring. After 15 min 2 drops of DMF were added. The reaction was vigorous with gas evolution. The mixture was stirred at room temperature until the solid was completely dissolved (about 2 h). The solution was evaporated. The crude material was re-dissolved in CH₂Cl₂ (150 ml) and slowly dropped into a suspension of K₂CO₃ (47 g) and (S)-1-cyclohexylethyl amine (29 ml, 0.196 mol) in CH₂Cl₂ (250 ml) maintaining the temperature between 10-15°C. The dark solution was left 1 h at room temperature and 1 h refluxing. The organic phase was then washed with water, NaOH 1N, brine, dried over Na₂SO₄ and then evaporated under vacuum. The crude residue was triturated with EtOAc. After filtration the title compound was obtained, mp = 177-180°C.
MW = 372.51
[α]_{D} = +21.77 (c = 0.4 in MeOH).

### Description 3. ((S)-1-Cyclohexylethyl)-3-bromomethyl-2-phenylquinoline-4-carboxamide

3-Methyl-2-phenyl-quinoline-4-carboxylic acid ((S)-1-cyclohexyl-ethyl)-amide (9.8 g, 26 mmol; compound prepared as in Description 2) and N-bromosuccinimmide (9.8 g, 55 mmol) were suspended in CCl₄ (100 ml) and warmed to incipient reflux. Dibenzoyl peroxide (about 300 mg) was carefully added dropwise and the solution was then refluxed for 2 h. The solvent was removed under vacuum and the residue was re-dissolved in CH₂Cl₂ (200 ml) and filtered. DCM was then evaporated and the residue was dissolved in EtOAc and washed with a saturated solution of NaHCO₃, brine, dried over Na₂SO₄, filtered and evaporated to give the title compound. The title compound may be used in the next step without further purification, mp: 182-184°C.
MW = 451.41
[α]_{D} = -5.76 (c= 0.5% in CH₂Cl₂)

### Description 4. 6,7-Difluoro-3-methyl-2-phenyl-quinoline-4-carboxylic acid.

A solution of 5,6-difluoroisatine (4.68 g; 25.6 mmol) (prepared as in JACS **1958**, *23*, 1858) and phenylethylketone (3.40 ml; 25.6 mmol) in glacial acetic acid (150 ml) was stirred for 5 minutes at 105°C. HCl 37% was added (38 ml) and the reaction mixture was stirred at 105°C for 24h. The reaction was then cooled at room temperature and diluted with water (400 ml). Filtration and subsequent drying of the precipitate afforded the title compound as a solid.

### Description 5. 3-(3-Oxo-piperazin-1-ylmethyl)-2-phenyl-quinoline-4-carboxylic acid ((S)-1-cyclohexyl-ethyl)-amide

A solution of 3-bromomethyl-2-phenyl-quinoline-4-carboxylic acid ((S)-1-cyclohexylethyl)-amide (10 g, 22 mmol, prepared as in Description 3), piperazin-2-one (CAS [5625-67-2]) (3 g, 30 mmol) and ethyldiisopropylamine (11 ml, 66 mmol) in dry THF (200 ml) was stirred for 24 h at room temperature. The solvent was evaporated to dryness in vacuum and the residue was re-dissolved in EtOAc. This mixture was washed with a dilute NaOH solution, with water and dried over Na₂SO₄. After evaporating to dryness, the residue was triturated with Et₂O to afford the desired compound, which may be used without further purification.

### Description 6. General procedure for piperazinone alkylation

Method A: To a solution of the piperazinone derivative (1 mmol, compound prepared as in Description 5) in anhydrous DMF (10 ml), 60% NaH (2 mmol, 29 mg) was added at 0°C. The dark solution obtained was stirred for 10 minutes at 0°C and then for additional 20 minutes at room temperature. The solution was re-cooled at 0°C and the electrophilic species (1 mmol) were added.
The reaction was stirred overnight then poured into a saturated solution of NaCl and extracted with EtOAc. The organic phases were dried over Na₂SO₄, filtered and evaporated under vacuum to give after purification by flash chromatography the desired compound.

Method B: A mixture of the piperazinone derivative (0.47g, 1 mmol, compound prepared as in Description 5), alkylating reagent (1.2 mmol) and KOH (0.23g, 4 mmol) in anhydrous DMSO (10 ml), was stirred for 36 hours at room temperature. The reaction was diluted with a saturated solution of NaCl and the product was extracted with DCM. The organic phases were dried over Na₂SO₄, filtered and evaporated under vacuum; the residue was purificated by flash chromatography to afford the desired compound.

### Description 7. 3-Bromomethyl-6,7-difluoro-2-phenyl-quinoline-4-carboxylic acid ((S)-1-cyclohexyl-ethyl)-amide

The compound was prepared according to Descriptions 2 and 3 starting from the compound in Description 4.

### Description 8. 3-Bromomethyl-6-fluoro-2-phenyl-quinoline-4-carboxylic acid ((S)-1-cyclohexyl-ethyl)-amide

The compound was prepared according to Description 1, starting from 5-fluoroisatine CAS[443-69-6] and phenylethylketone, Description 2 and 3.

### Description 9. 6-Fluoro-3-(3-oxo-piperazin-1-ylmethyl)-2-phenyl-quinoline-4-carboxylic acid ((S)-1-cyclohexyl-ethyl)-amide

The compound was prepared according to Description 5 starting from the compound of Description 8 and piperazinone (CAS[5625-67-2]).

### Description 10. 3-Bromomethyl-2-thiophen-2-yl-quinoline-4-carboxylic acid ((S)-1-cyclohexyl-ethyl)-amide

The compound was prepared according to Description 1, starting from 1-thiophen-2-yl-propan-1-one CAS [13679-75-9] and isatine, Description 2 and 3.

### Description 11. 3-(3-Oxo-piperazin-1-ylmethyl)-2-thiophen-2-yl-quinoline-4-carboxylic acid ((S)-1-cyclohexyl-ethyl)-amide

The compound was prepared according to Description 5 starting from the compound of Description 10 and piperazinone (CAS [5625-67-2]).

### Description 12. 3-Bromomethyl-2-thiophen-3-yl-quiaoline-4-carboxylic acid ((S)-1-cyclo6exyf-ethyl)-amide

The compound was prepared according to Description 1, starting from 1-thiophen-3-yl-propan-1-one CAS [51179-52-3] and isatine, Description 2 and 3.

### Description 13. 4-[4-((S)-1-Cyclohexyl-ethylcarbamoyl)-6-fluoro-2-phenyl-quinolin-3-ylmethyl]-3-oxo-piperazine-1-carboxylic acid tert-butyl ester

To a suspension of 3-oxo-piperazine-1-carboxylic acid tert-butyl ester (0.3 g, 1.5 mmol, CAS [76003-29-7]) in DMF/DMSO 2/1 (10 ml), 60% NaH (60 mg, 1.5 mmol) was added at 0°C. After stirring for 30 minutes a solution of 3-bromomethyl-6-fluoro-2-phenylquinoline-4-carboxylic acid ((S)-1-cyclohexyl-ethyl)-amide (0.6 g, 1.3 mmol, prepared according to Description 8) in DMF (3ml) was slowly added. The reaction was stirred for 3 hours at room temperature then poured into a saturated solution of NaCl. The precipitate was filtered and purified by flash chromatography to afford the title compound.

### Description 14: Piperazine-1-carboxylic acid tert-butyl ester.

To a solution of piperazine (30 g, 350 mmol) in water (370 ml) and tBuOH (420 ml), a solution of 4N NaOH (70 ml) was added. The mixture was cooled to 0°C and then BOC₂O (38 g, 170 mmol) was added portionwise. After stirring at room temperature for 45 minutes, tBuOH was evaporated under vacuum, the precipitate (diBOCpiperazine) was filtered and water was extracted with CH₂Cl₂. After drying over Na₂SO₄ the solvent was removed under vacuum to afford the title compound.
MW = 186.25

### Description 15: 2-Phenyl-3-piperazin-1-ylmethyl-quinoline-0-carboxylic acid ((S)-1-cyclohexyl-ethyl)-amide.

4-[4-((S)-1-Cyclohexyl-ethylcarbarnoyl)-2-phenyl-quinolin-3-ylmethyl]-piperazine-1-carboxylic acid tert-butyl ester (2.5 g, 4.5 mmol, prepared according to Description 5 using piperazine-1-carboxylic acid tert-butyl ester (Description 14) as nucleophile) was dissolved in CH₂Cl₂ (60 ml) and TFA (3 ml) was added. The red solution was stirred at room temperature overnight; then the solvent and the excess of TFA were removed under vacuum. The residue was dissolved in H₂O and washed 2 times with Et₂O. The water extract was made alkaline by addition of 2N NaOH solution and the product was extracted with EtOAc. The solvent was evaporated to dryness and the residue was purified by flash chromatography (eluent CH₂Cl₂: MeOH 93:7) to afford the title compound.

### Description 16. General procedure for synthesis of ureas

A solution of 2-phenyl-3-piperazin-1-ylmethyl-quinoline-4-carboxylic acid ((S)-1-cyclohexyl-ethyl)-amide (0.3 g, 0.65 mmol, prepared according to Description 15), isocyanate (0.65 mmol) in CH₃CN (20 ml) was stirred for 1 hour at room temperature. The solvent was evaporated under vacuum; the residue was re-dissolved in EtOAc and washed with a saturated solution of NaCl. The organic layer was dried over Na₂SO₄, filtered and evaporated. The residue was purified by column chromatography to afford the urea derivative.

### Examples from 1 to 15, 17 and 22, in Table 1, were prepared according to the following general procedure:

A solution of 3-bromomethyl-2-phenyl-quinoline-4-carboxylic acid ((S)-1-cyclohexylethyl)-amide (1 mmol, 0.45 g; compound prepared as in Description 3), 1.5 mmol of amine and ethyldiisopropylamine (3 mmol, 0.5 ml) in dry THF (15 ml) was stirred for 24 h at room temperature. The solvent was evaporated to dryness in vacuum and the residue was re-dissolved in EtOAc. This mixture was washed with a dilute NaOH solution, with water and dried over Na₂SO₄. After evaporating to dryness, the residue was purified by flash chromatography to afford the desired compound.

### Example 16. 6-Fluoro-3-[3-oxo-4-(2-piperidin-1-yl-ethyl)-piperazin-1-ylmethyl]-2-phenyl-quinoline-4-carboxylic acid ((S)-1-cyclohexyl-ethyl)-amide.

The compound was prepared following the Description 6 (method A) starting from the compound of Description 9 and 1-(2-chloroethyl)piperidine.

### Example 18. 3-[3-Oxo-4-(3-piperidin-1-yl-propyl)-piperazin-1-ylmethyl]-2-phenylquinoline-4-carboxylic acid ((S)-1-cyclohexyl-ethyl)-amide.

The compound was prepared following the Description 6, method A, starting from the compound of Description 5 and 1-3-(chloropropyl)piperidine (CAS[5472-49-1]).

### Example 19. 3-(1-Oxo-3,4-dihydro-1H-pyrrolo[1,2-a]pyrazin-2-ylmethyl)-2-phenylquinoline-4-carboxylic acid (1-cyclohexyl-ethyl)-amide.

The compound was prepared according to Description 13 using 3,4-dihydro-2H-pyrrolo[1,2-a]pyrazin-1-one (CAS[54906-42-2]) and compound of Description 3.

### Example 20. 3-Dimethylaminomethyl-6-fluoro-2-phenyl-quinoline-4-carboxylic acid ((S)-1-cyclohexyl-ethyl)-amide.

The compound was prepared according to Description 5 starting from the compound in Description 8 and dimethylamine.

### Example 21. 6-Fluoro-3-(1-oxo-3,4-dihydro-1H-pyrrolo[1,2-a]pyrazin-2-ylmethyl)-2-phenyl-quinoline-4-carboxylic acid ((S)-1-cyclohexyl-ethyl)-amide.

The compound was prepared according to Description 13 starting from the compound of Description 8 and 3,4-dihydro-2H-pyrrolo[1,2-a]pyrazin-1-one (CAS[54906-42-2]).

### Example 23. 3-[4-(3-Dimethylamino-propyl)-3-oxo-giperazin-1-ylmethylJ-2-phenylquinoline-4-carboxylic acid ((S)-1-cyclohexyl-ethyl)-amide.

The compound was prepared following Description 6, method B, starting from the compound prepared in Description 5 and 3-dimethylaminopropylchloride.

### Example 24. 3-(4-Methyl-3-oxo-piperazin-1-ylmethyl)-2-phenyl-quinoline-4-carboxylic acid ((S)-1-cyclohexyl-ethyl)-amide ditrifluoroacetate.

The compound was prepared following Description 6, method A, starting from the compound of Description 5 and methyl iodide.

### Example 25. 3-(4-Ethyl-3-oxo-piperazin-1-ylmethyl)-2-phenyl-quinoline-4-carboxylic acid ((S)-1-cyclohexyl-ethyl)-amide.

The compound was prepared following the above Description 6, method A, starting from the compound in Description 5 and ethyl bromide.

### Example 26. 3-(2-Oxo-imidazolidin-1-ylmethyl)-2-phenyl-quinoline-4-carboxylic acid ((S)-1-cyclohexyl-ethyl)-amide.

A mixture of 3-bromomethyl-2-phenyl-quinoline-4-carboxylic acid ((S)-1-cyclohexylethyl)-amide (0.5g, 1.1 mmol) (prepared as in Description 3), 2-imidazolidone (0.1g, 1.2 mmol, and K₂CO₃ (0.3 g, 2.2 mmol) in CH₃CN (20 ml) was stirred overnight at room temperature. The carbonate was filtered and the solvent evaporated under vacuum. The solid was re-dissolved in EtOAc, washed with 0.5 N NaOH and a saturated solution of NaCl. The organic layer was dried over Na₂SO₄, filtered and evaporated to dryness. The residue was purified on column chromatography (CH₂Cl₂/MeOH 95/5) to give the title compound.

### Example 27. 3-[3-Oxo-4-(2-pyrrolidin-1-yl-ethyl)-piperazin-1-ylmethyl]-2-phenylquinoline-4-carboxylic acid ((S)-1-cyclohexyl-ethyl)-amide.

The compound was prepared following Description 6 (method B) starting from the compound of Description 5 and 1-(2-chloroethyl)pyrrolidine.

### Example 28. 3-[4-(2-Diethylamino-ethyl)-3-oxo-piperazin-1-ylmethyl]-2-phenylquinoline-4-carboxylic acid ((S)-1-cyclohexyl-ethyl)-amide.

The compound was prepared following Description 6 (method B) starting from the compound of Description 5 and 2-diethylaminoethyl chloride.

### Example 29. 3-[4-(2-Dimethylamino-ethyl)-3-oxo-piperazin-1-ylmethyl]-2-phenylquinoline-4-carboxylic acid ((S)-1-cyclobexyl-etbyl)-amide.

The compound was prepared following Description 6 (method B) starting from the compound in Description 5 and dimethylamino ethyl chloride.

### Example 30. 3-[4-(2-Amino-ethyl)-3-oxo-piperazin-1-ylmethyl]-2-phenyl-quinoline-4-carboxylic acid ((S)-1-cyclobexyl-ethyl)-amide.

To a solution of (2-{4-[4-((S)-1-cyclohexyl-ethylcarbamoyl)-2-phenyl-quinolin-3-ylmethyl]-2-oxo-piperazin-1-yl}-ethyl)-carbamic acid tert-butyl ester (30 mg, 0.05 mmol, prepared according to Description 6 (method B) starting from the compound in Description 5 and 2-(BOC-amino)ethylbromide) in CH₂Cl₂ (4 ml), TFA (0.2 ml) was added drop-wise at room temperature. Stirring was continued for 1 hour. The solvent was evaporated under vacuum and the residue was basified with a saturated solution of K₂CO₃ and extracted with ethyl acetate. The organic layer was dried over Na₂SO₄, filtered and evaporated to give the title compound.

### Example 31. 3-[4-(2-Morpholin-4-yl-ethyl)-3-oxo-piperazin-1-ylmethyl]-2-phenylquinoline-4-carboxylic acid ((S)-1-cyclohexyl-ethyl)-amide.

The compound was prepared following Description 6 (method B) starting from the compound in Description 5 and 4-(2-chloroethyl)morpholine.

### Example 32. 3-(4-Ethylcarbamoyl-piperazin-1-ylmethyl)-2-phenyl-quinoline-4-carboxylic acid ((S)-1-cyclohexyl-ethyl)-amide.

The compound was prepared following Description 16 starting from compound in Description 15 and ethylisocyanate.

### Example 33. 3-(4-Isopropylcarbamoyl-piperazin-1-ylmethyl)-2-phenyl-quinoline-4-carboxylic acid ((S)-1-cyclohexyl-ethyl)-amide

The compound was prepared following Description 16 starting from the compound of Description 15 and isopropylisocyanate.

### Example 34. 3-(3-Oxo-piperazin-1-ylmethyl)-2-thiophen-3-yl-quinoline-4-carboxylic acid ((S)-1-cyclohexyl-ethyl)-amide.

The compound was prepared according to Description 5 starting from the compound of Description 12 and piperazinone (CAS [5625-67-2]).

### Example 35. 3-[4-(3-Dimethylamino-propyl)-3-oxo-piperazin-1-ylmethyl]-6-fluoro-2-phenyl-quinoline-4-carboxylic acid ((S)-I-cyciohexyl-ethyl)-amide.

The compound was prepared following Description 6, method A, starting from the compound of Description 9 and 3-dimethylaminopropylchloride.

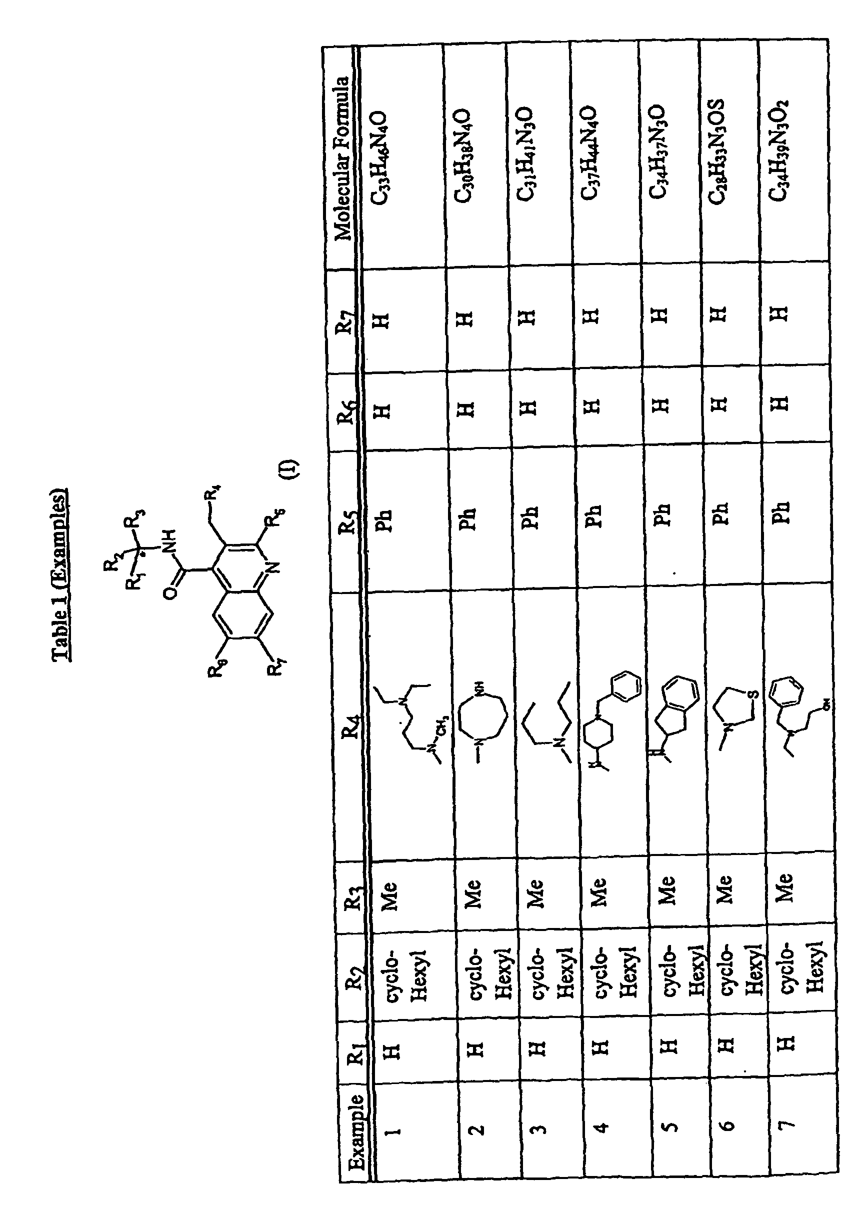

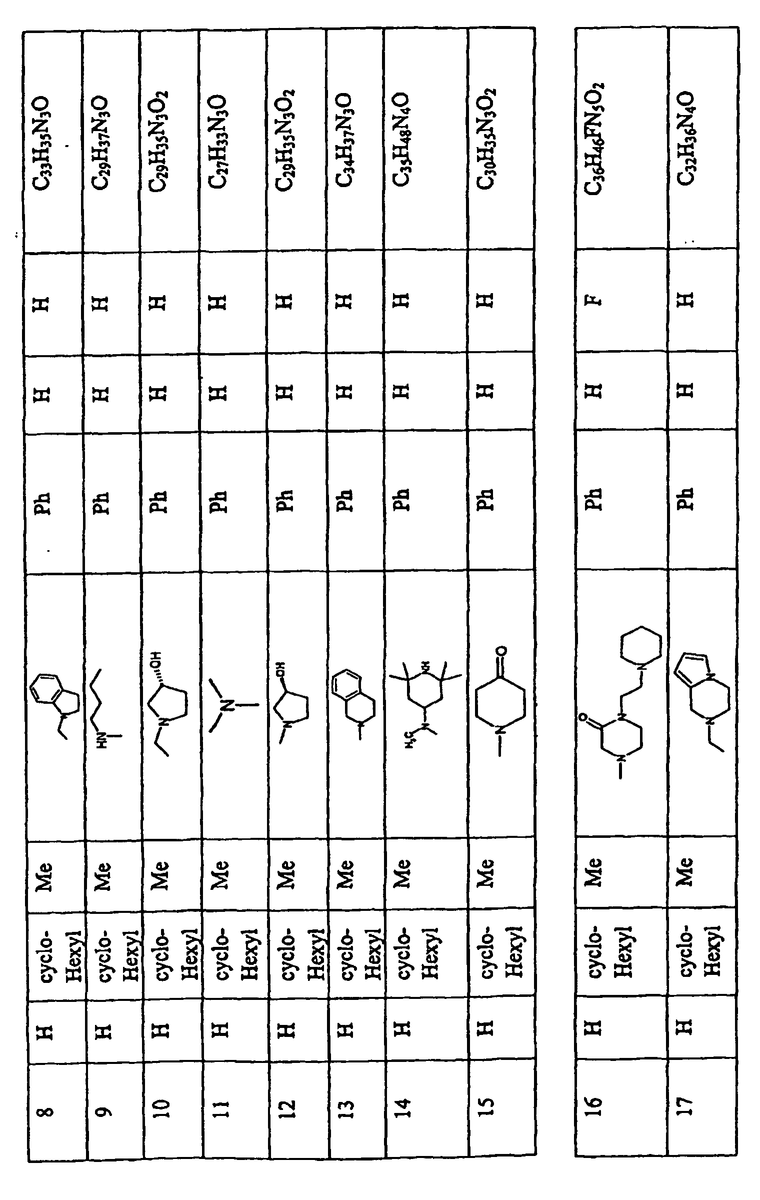

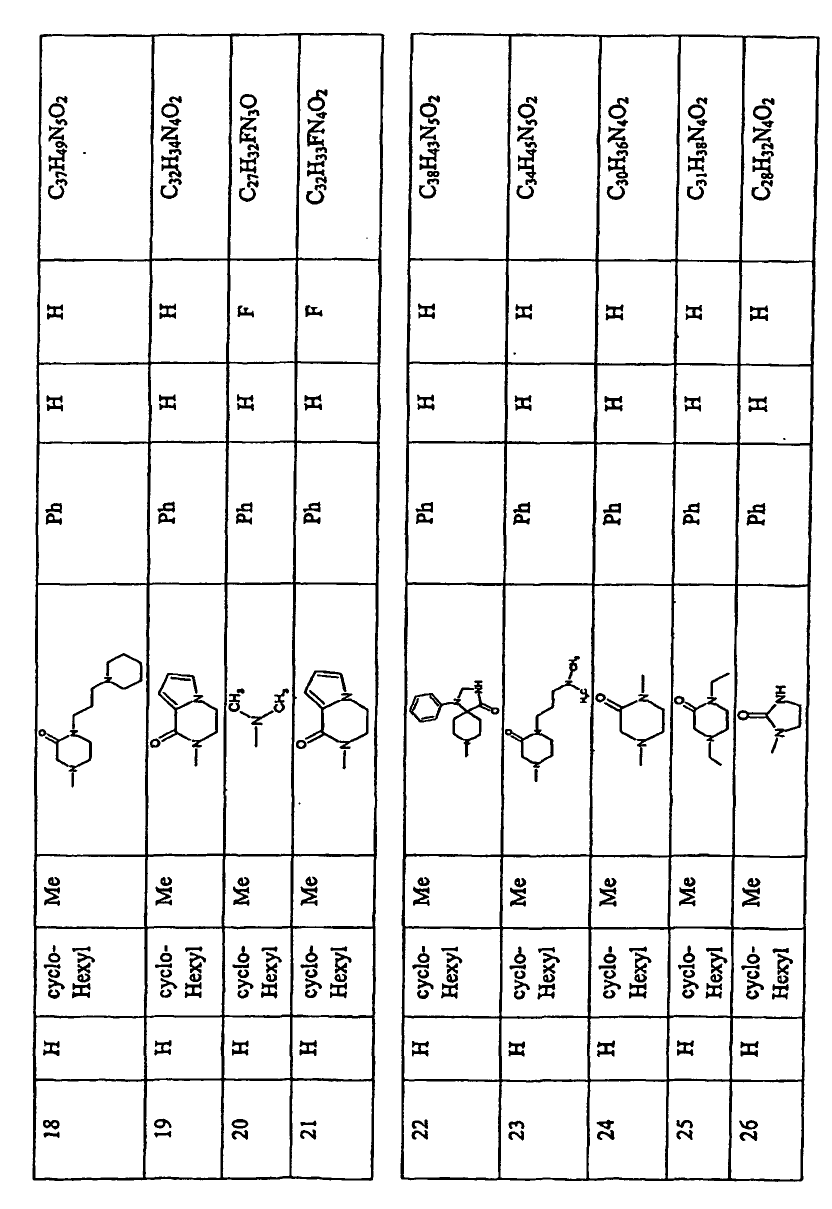

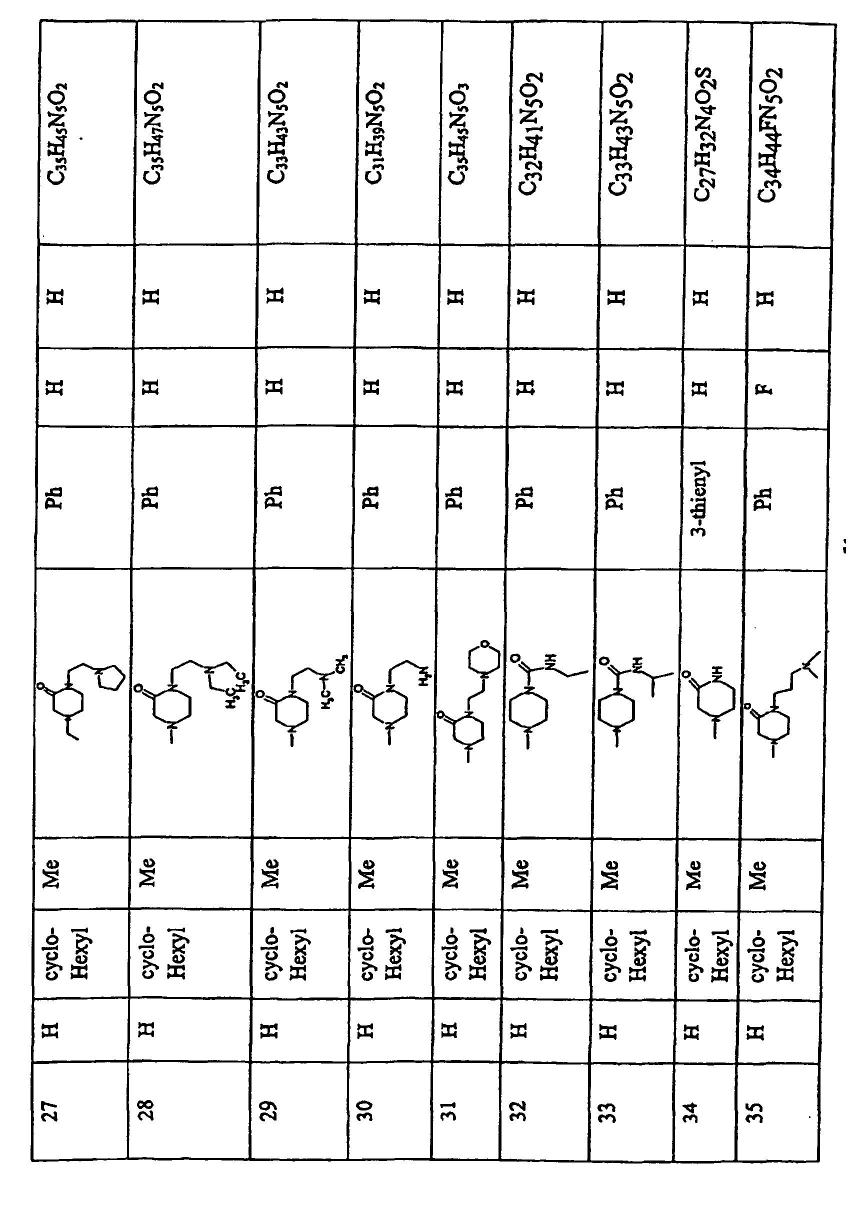

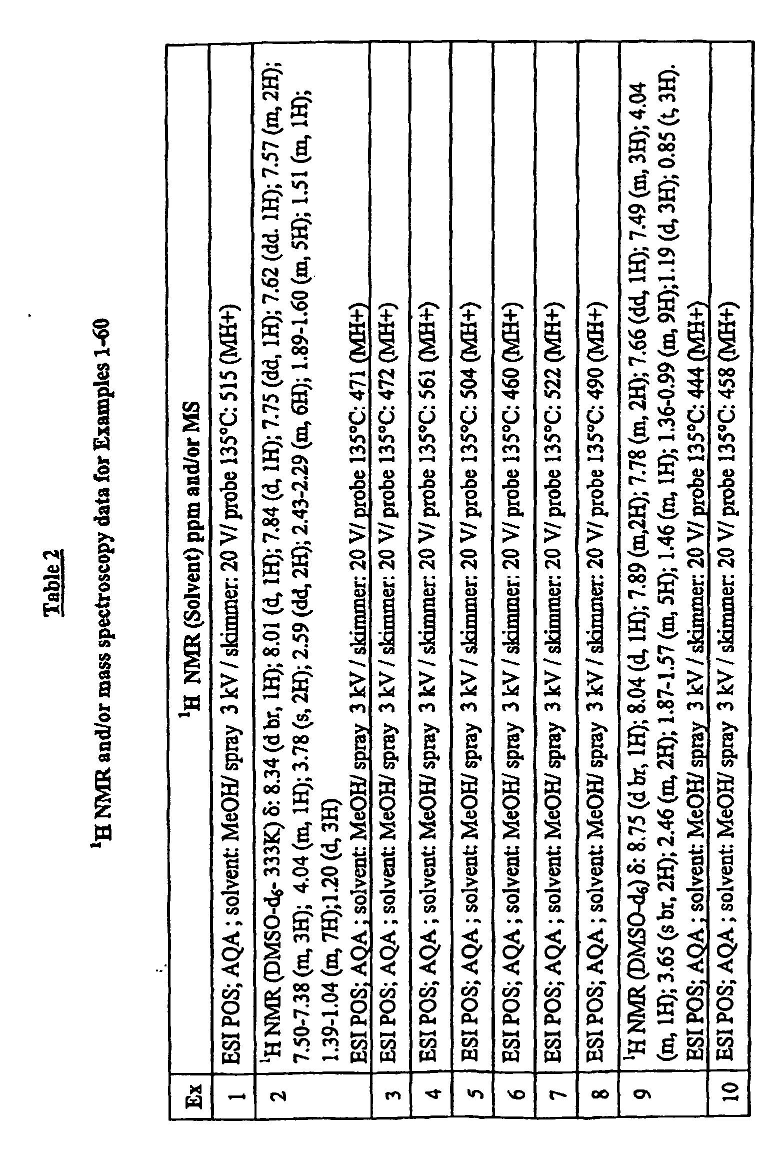

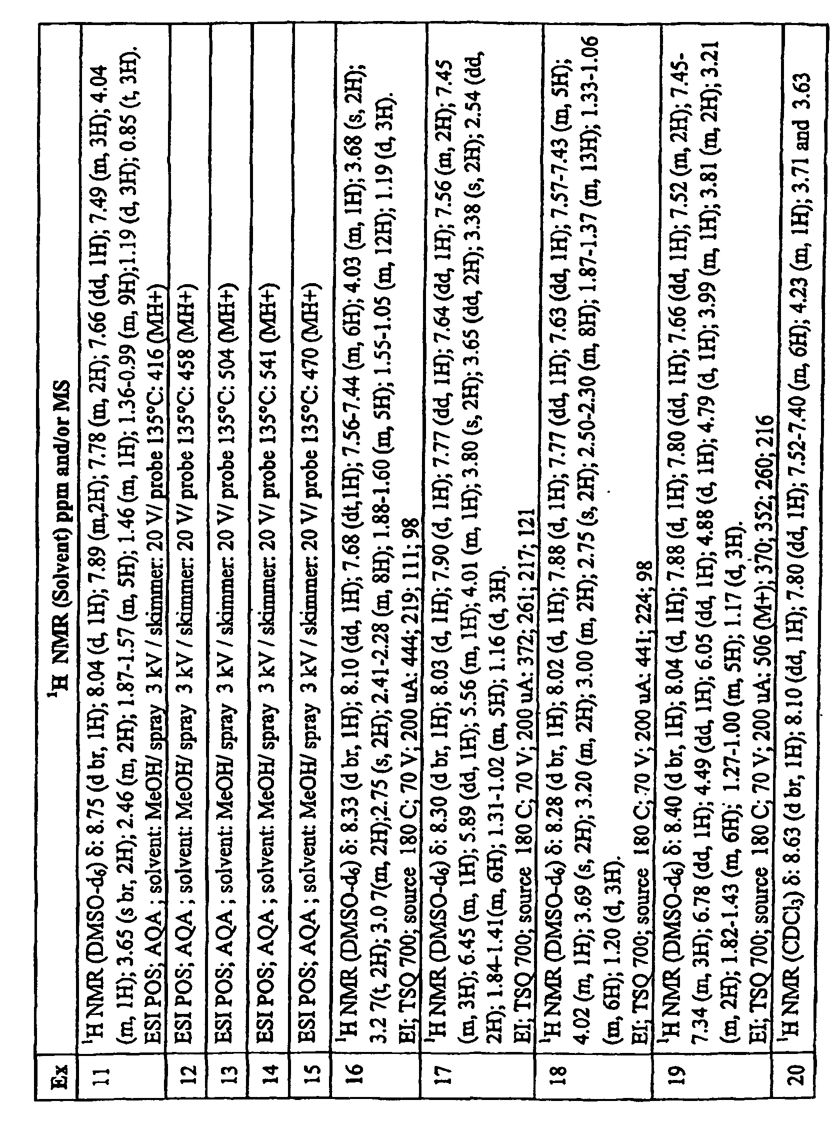

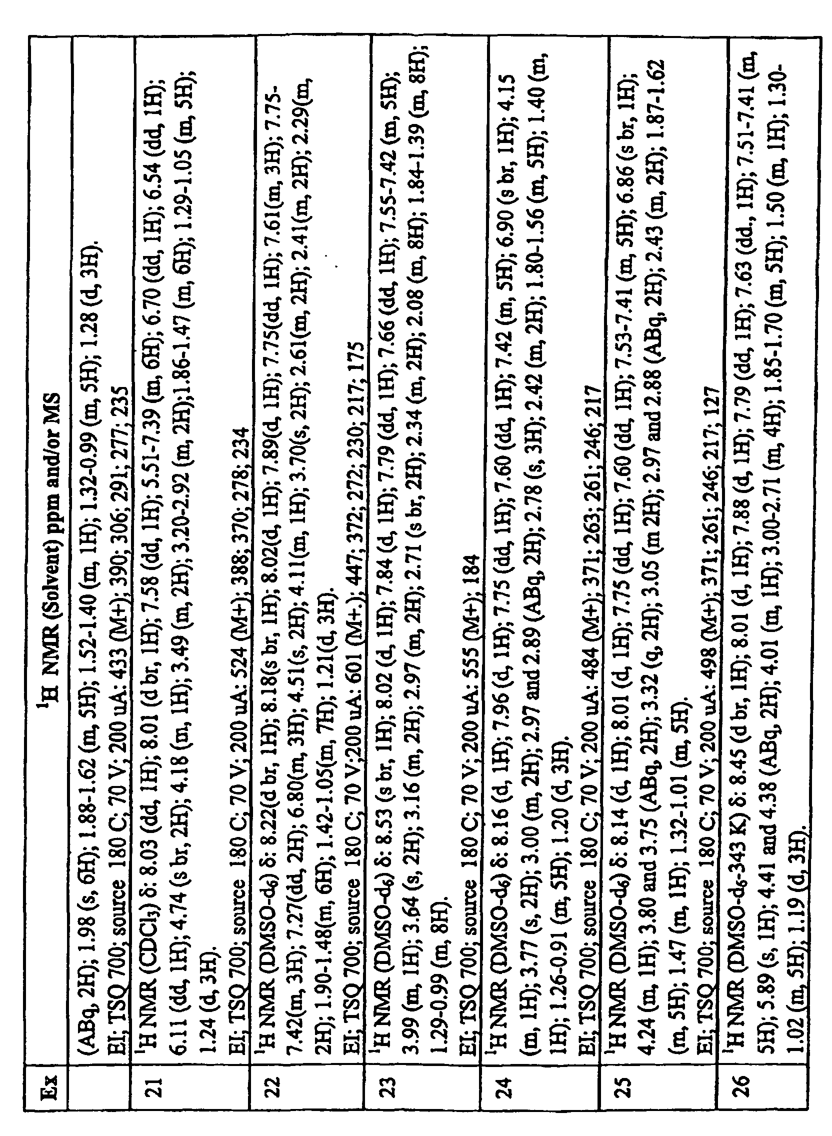

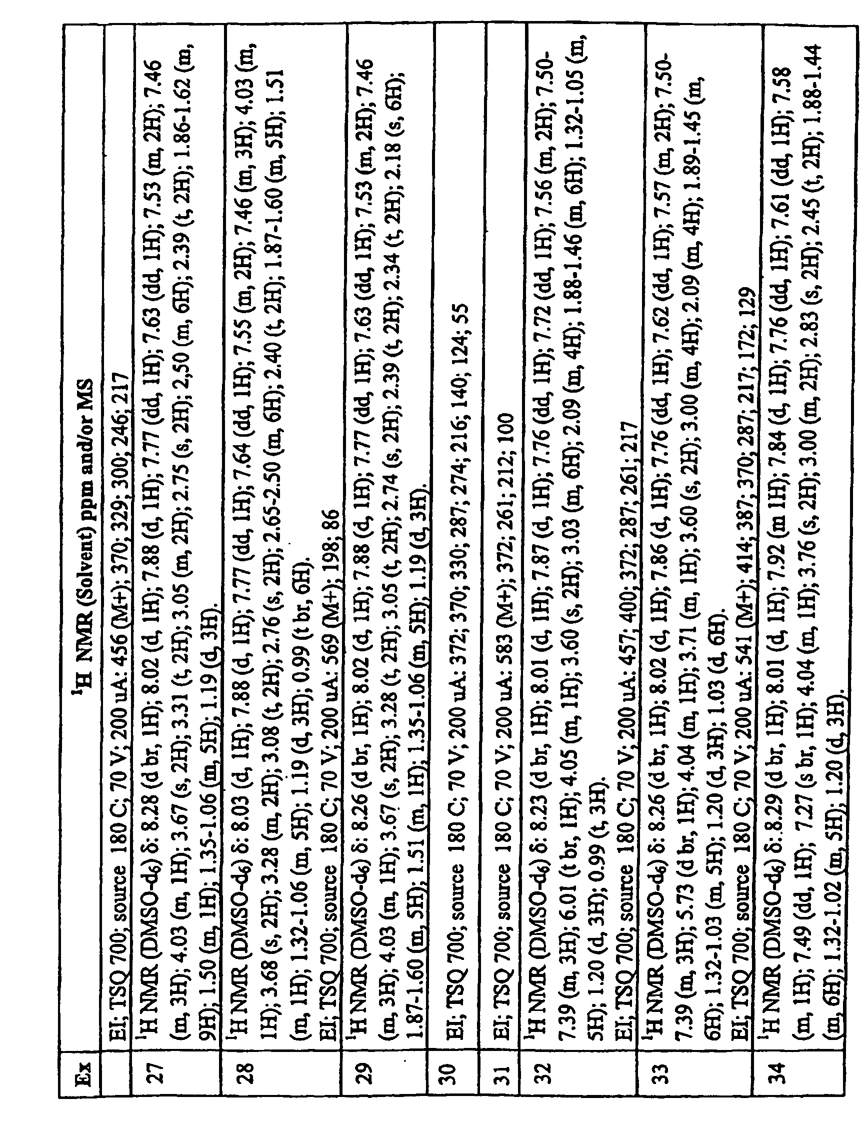

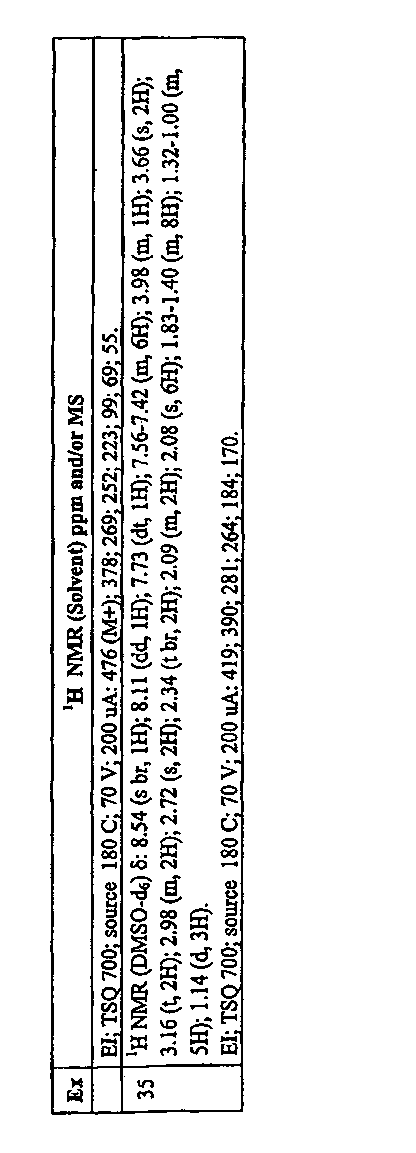

**Table 3**

| **Melting points (Mp/°C) and/or Refractory indices ([α]**_{**D**}^{**20**}**) of certain Examples of Table 1** | | |
|---|---|---|
| **Example Number** | **Mp (°C)** | **[α]**_{**D**}^{**20**} |
| 2 | 148-150 | +4.27 (c=0.5%. MeOH) |
| 9 | 130-134 | -7.67 (c=0.1%, MeOH) |
| 16 | 147-149 | +13.71 (c=0.5%, MeOH) |
| 17 | 170-175 | +20.66 (c=0.125%, MeOH) |
| 20 | 188-190 | - |
| 21 | 120-130 | +23.24 (c=0.11%, MeOH) |
| 22 | >250 | +26.03 (c=0.125%, MeOH) |
| 23 | 148-150 | -39.74 (c=0.1%, MeOH) |
| 24 | 190-192 | +16.59 (c=0.1%, MeOH) |
| 25 | 229-231 | +13.6 (c=0.1%, MeOH) |
| 26 | 185 | - |
| 32 | 150 | +14.85 (c=0.2%, MeOH) |
| 33 | 176 | 14.64 (c=0.2%, MeOH) |
| 34 | 181-184 | +10.52 (c=0.05%, MeOH) |
| 35 | 110-115 | +4.47 (c=0.5%. MeOH) |

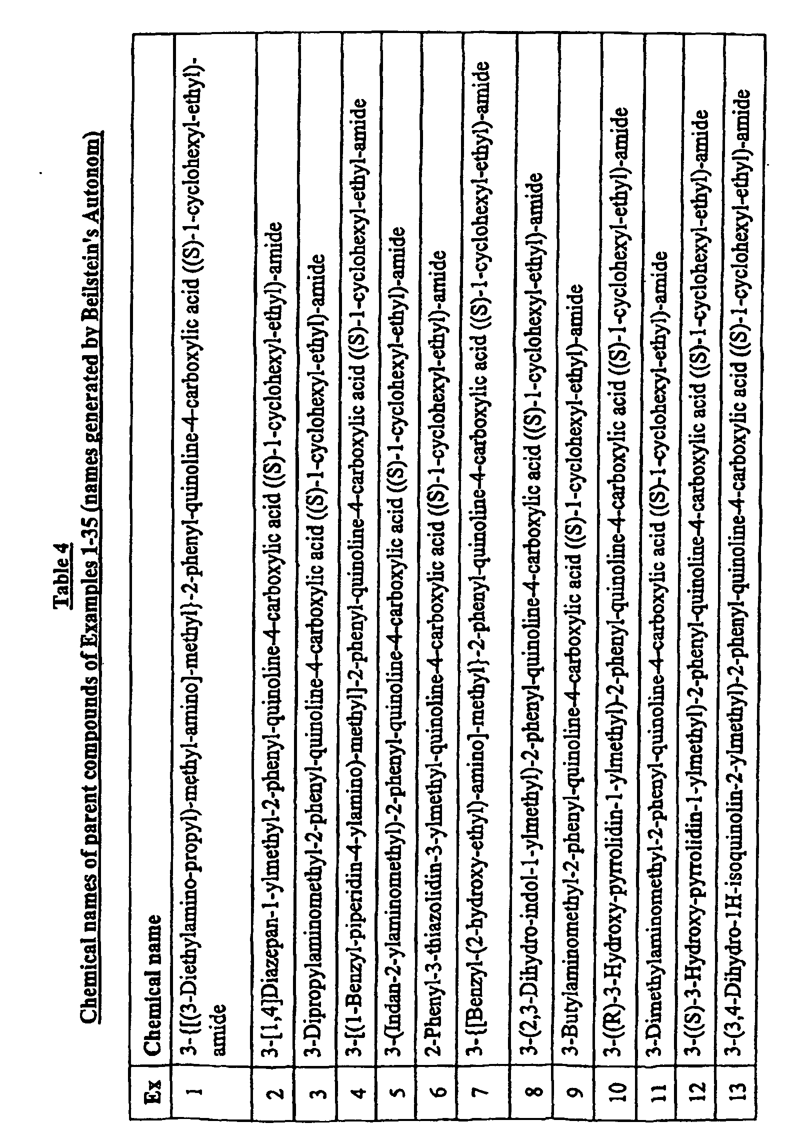

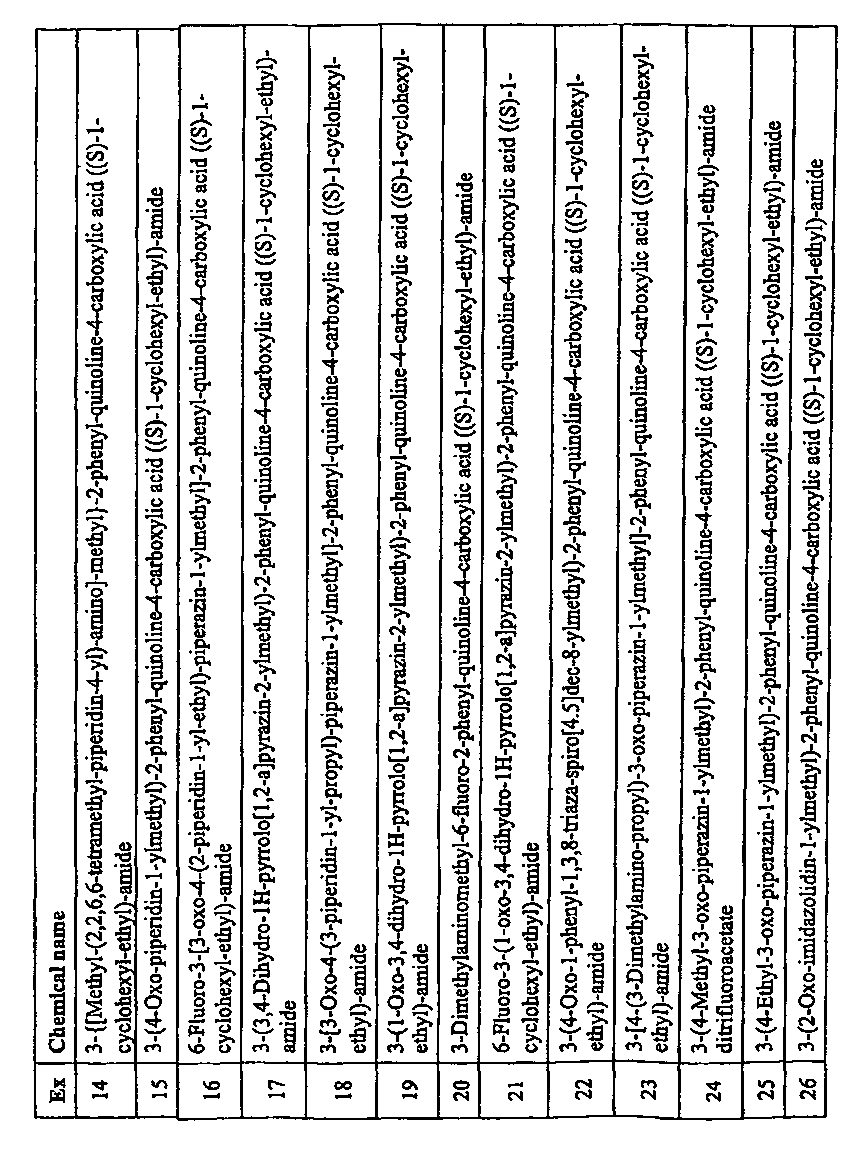

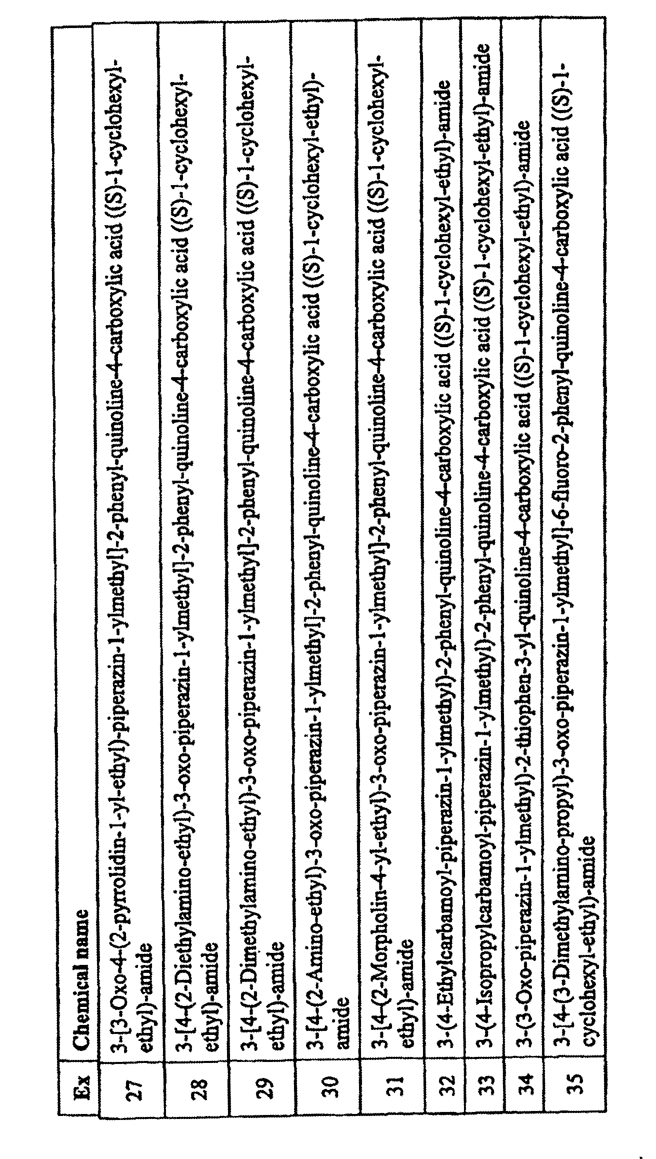

## Claims

1. A tachykinin receptor antagonist selected from the group consisting of:
3- {[(3-Diethylamino-propyl)-methyl-amino]-methyl}-2-phenyl-quinoline-4-carboxylic acid ((S)-1-cyclohexyl-ethyl)-amide;
3-[1,4]Diazepan-1-ylmethyl-2-phenyl-quinoline-4-carboxylic acid ((S)-1-cyclohexylethyl)-amide;
3-Dipropylaminomethyl-2-phenyl-quinoline-4-carboxylic acid ((S)-1-cyclohexyl-ethyl)-amide;
3-[(1-Benzyl-piperidin-4-ylamino)-methyl]-2-phenyl-quinoline-4-carboxylic acid ((S)-1-cyclohexyl-ethyl-amide;
3-(Indan-2-ylaminomethyl)-2-phenyl-quinoline-4-carboxylic acid ((S)-1-cyclohexylethyl)-amide;
2-Phenyl-3-thiazolidin-3-ylmethyl-quinoline-4-carboxylic acid ((S)-1-cyclohexyl-ethyl)-amide;
3-{[Benzyl-(2-hydroxy-ethyl)-amino]-methyl}-2-phenyl-quinoline-4-carboxylic acid ((S)-1-cyclohexyl-ethyl)-amide;
3-(2,3-Dihydro-indol-1-ylmethyl)-2-phenyl-quinoline-4-carboxylic acid ((S)-1-cyclohexyl-ethyl)-amide;
3-Butylaminomethyl-2-phenyl-quinoline-4-carboxylic acid ((S)-1-cyclohexyl-ethyl)-amide;
3-((R)-3-Hydroxy-pyrrolidin-1-ylmethyl)-2-phenyl-quinoline-4-carboxylic acid ((S)-1-cyclohexyl-ethyl)-amide;
3-Dimethylaminomethyl-2-phenyl-quinoline-4-carboxylic acid ((S)-1-cyclohexyl-ethyl)-amide;
3-((S)-3-Hydroxy-pyrrolidin-1-ylmethyl)-2-phenyl-quinoline-4-carboxylic acid ((S)-1-cyclohexyl-ethyl)-amide;
3-(3,4-Dihydro- 1H-isoquinolin-2-ylmethyl)-2-phenyl-quinoline-4-carboxylic acid ((S)-1-cyclohexyl-ethyl)-amide;
3-{[Methyl-(2,2,6,6-tetramethyl-piperidin-4-yl)-amino]-methyl}-2-phenyl-quinoline-4-carboxylic acid ((S)-1-cyclohexyl-ethyl)-amide;
3-(4-Oxo-piperidin-I-ylmethyl)-2-phenyl-quinoline-4-carboxylic acid ((S)-1-cyclohexylethyl)-amide;
6-Fluoro-3-[3-oxo-4-(2-piperidin-1-yl-ethyl)-piperazin-1-ylmethyl]-2-phenyl-quinoline-4-carboxylic acid ((S)-1-cyclohexyl-ethyl)-amide;
3-(3,4-Dihydro-1H-pyrrolo[1,2-a]pyrazin-2-ylmethyl)-2-phenyl-quinoline-4-carboxylic acid ((S)-1-cyclohexyl-ethyl)-amide;
3-[3-Oxo-4-(3-piperidin-1-yl-propyl)-piperazin-1-ylmethyl]-2-phenyl-quinoline-4-carboxylic acid ((S)-1-cyclohexyl-ethyl)-amide;
3-(1-Oxo-3,4-dihydro-1H-pyrrolo[1,2-a]pyrazin-2-ylmethyl)-2-phenyl-quinoline-4-carboxylic acid ((S)-1-cyclohexyl-ethyl)-amide;
3-Dimethylaminomethyl-6-fluoro-2-phenyl-quinoline-4-carboxylic acid ((S)-1-cyclohexyl-ethyl)-amide;
6-Fluoro-3-(1-oxo-3,4-dihydro-1 H-pyrrolo[1,2-a]pyrazin-2-ylmethyl)-2-phenyl-quinoline-4-carboxylic acid ((S)-1-cyclohexyl-ethyl)-amide;
3-(4-Oxo-1-phenyl-1,3,8-triaza-spiro[4.5]dec-8-ylmethyl)-2-phenyl-quinoline-4-carboxylic acid ((S)-1-cyclohexyl-ethyl)-amide;
3-[4-(3-Dimethylamino-propyl)-3-oxo-piperazin-1-ylmethyl]-2-phenyl-quinoline-4-carboxylic acid ((S)-1-cyclohexyl-ethyl)-amide;
3-(4-Methyl-3-oxo-piperazin-1-ylmethyl)-2-phenyl-quinoline-4-carboxylic acid ((S)-1-cyclohexyl-ethyl)-amide ditrifluoroacetate;
3-(4-Ethyl-3-oxo-piperazin-1-ylmethyl)-2-phenyl-quinoline-4-carboxylic acid ((S)-1-cyclohexyl-ethyl)-amide;
3-(2-Oxo-imidazolidin-1-ylmethyl)-2-phenyl-quinoline-4-carboxylic acid ((S)-1-cyclohexyl-ethyl)-amide;
3-[3-Oxo-4-(2-pyrrolidin-1-yl-ethyl)-piperazin-1-ylmethyl]-2-phenyl-quinoline-4-carboxylic acid ((S)-1-cyclobexyl-ethyl)-unide;
3-[4-(2-Diethylamino-ethyl)-3-oxo-piperazin-1-ylmethyl]-2-phenyl-quinoline-4-carboxylic acid ((S)-1-cyclohexyl-ethyl)-amide;
3-[4-(2-Dimethylamino-ethyl)-3-oxo-piperazin-1-ylmethyl]-2-phenyl-quinoline-4-carboxylic acid ((S)-1-cyclohexyl-ethyl)-amide;
3-[4-(2-Amino-ethyl)-3-oxo-piperazin-1-ylmethyl]-2-phenyl-quinoline-4-carboxylic acid ((S)-1-cyclohexyl-ethyl)-amide;
3-[4-(2-Morpholin-4-yl-ethyl)-3-oxo-piperazin-1-ylmethyl]-2-phenyl-quinoline-4-carboxylic acid ((S)-1-cyclohexyl-ethyl)-amide;
3-(4-Ethylcarbamoyl-piperazin-1-ylmethyl)-2-phenyl-quinoline-4-carboxylic acid ((S)-1-cyclohexyl-ethyl)-amide;
3-(4-Isopropylcarbamoyl-piperazin-1-ylmethyl)-2-phenyl-quinoline-4-carboxylic acid ((S)-1-cyclohexyl-ethyl)-amide;
3-(3-Oxo-piperazin-1-ylmethyl)-2-thiophen-3-yl-quinoline-4-carboxylic acid ((S)-1-cyclohexyl-ethyl)-amide; and
3-[4-(3-Dimethylamino-propyl)-3-oxo-piperazin-1-ylmethyl]-6-fluoro-2-phenylquinoline-4-carboxylic acid ((S)-1-cyclohexyl-ethyl)-amide; or a pharmaceutically acceptable salt or solvate thereof.

2. A pharmaceutical composition comprising a compound according to claim 1 and a pharmaceutically acceptable carrier.

3. A compound according to claim 1 for use as an active therapeutic substance.

4. A compound according to claim 1 for the treatment or prophylaxis of the Primary and Secondary Conditions of the invention.

5. Use of a compound according to claim 1 in the manufacture of a medicament for the treatment of the Primary and Secondary Conditions of the invention.

## Patentansprüche

1. Tachykinin-Rezeptorantagonist ausgewählt aus:
3-{[(3-Diethylaminopropyl)methylamino]methyl}-2-phenylchinolin-4-carbonsöre ((S)-1-cyclohexylethyl)amid;
3-[1,4]Diazepan-1-ylmethyl-2-phenylchinolin-4-carbonsäure((S)-1-cyclohexylethyl)-amid;
3-Dipropylaminomethyl-2-phenylehinolin-4-carbonsaure((S)-1-cyclobexylethyl)amid;
3-[(1-Benzylpiperidin-4-ylamino)methyl]-2-phenylchinolin-4-carbonsäure((S)-1-cyclohexylethyl)amid;
3-(Indan-2-ylaminomethyl)-2-phenylchinolin-4-carbozisäure-(S)-1-cyclohexylethyl)amid;
2-Phenyl-3-thiazolidin-3-ylmethylchinolin-4-carbonsaure((S)-1-cyclohexylethyl)amid
3-{[Benzyl-(2-hydroxyethyl)amino]methyl}-2-phenylchinolin-4-carbonsäure((S)-1-cyclob excylethyl)amid;
3-(2,3-)Dihydroindol-1-ylmethyl)-2-phenylehinolin-4-carboisäure((S)-1-cyclohexylethyl)amid;
3-Butylaminomethyl-2-Phenylchinolin-4-carbonsäure((S)-1-cyclohexylethyl)amid;
3-((R)-3-Hydroxypyrrolidin-1-ylmethyl)-2-phenylchinolin-4-carbonsäure((S)-1-cyclohexylethyl)amid;
3-Dimethylaminomethyl-2-phenylchinolin-4-carbonsäure((S)-1-cyclohexylethyl)amid;
3-((S)-3-Hydioxypyrrolidin-1-ylmethyl)-2-phenylchinolin-4-carbonsäure((S)-1-cyclohexylethyl)amid;
3-(3,4-Dihydro-1H-isochinolin-2-ylmethyl)-2-phenylchinolin-4-carbonsäure((S)-1. cyclohexylethyl)amid;
3- {[Methyl-(2,2,6,6-tetramethylpiperidin-4-yl)amino]methyl}-2-phenylchinolin-4-carbonsäure((S)-1-cyclohexylethyl)amid;
3-(4-Oxopiperidin-1-ylmethyl)-2-phenylchinolin-4-carbonsäure((S)-1-cyclohexylethyl)-amid;
6-Fluor-3-[3-oxo-(2-piperidin-1-ylethyl)piperazin-1-yl-methyl]-2-phenylchinolin-4-carbonsäure((S)-1-cyclophexylethyl)amid;
3-(3,4-Dihydro-1H-pyrrolo[1,2-a]pyrazin-2-ylmethyl)-2-phenylchinolin-4-carbonsäure-((S)-1-cyclohexylethyl)amid;
3-[3-Oxo-4-(3-piperidin-1-ylpropyl)piperazin-1-ylmethyl]-2-phenylchinolin-4-carbonsäure((S)-1-cyclohexylethyl)amid;
3-(1-Oxo-3,4-dihydro-1H-pyrrolo [1,2-a]pyrazin-2-ylmethyl)-2-phenylchinolin-4-carbonsäure((S)-1-cyctohexylethyl)amid,
3-Dimethylaminomethyl-6-fluor-2-phenylchinolin-4-carbonsäure((S)-1-cyclohexylethyl)-amid;
6-Fluor-3-(1-oxo-3,4-dihydro-1H-pyrrolo[1,2-a]pyrazin-2-ylmethyl)-2-phenylchinolin-4-carbonsäure((S)-1-cyclohexylethyl)amid;
3-(4-Oxo-1-phenyl-1,3,8-triaza-spiro[4.5]dec-8-ylmethyl)-2-phenylchinolin-4-carbonsäure((S)-1-cyclohexylethyl)amid;
3-[4-(3-Dimetylaminopropyl)-3-oxopiperazin-1-ylmethyl]-2-phenylchinolin-4-carbonsäure((S)-1-clyclohexylethyl)amid;
3-(4-Methyl-3-oxopiperazin-1-ylmethyl)-2-phenylchinolin-4-carbonsäure ((S) -cyclohexylethyl)amidditrifluoracetat;
3-(4-Ethyl-3-oxopiperazin-1-ylmethyl)-2-phenylchinolin-4-carbonsäure((S)-1-cyclohexylethyl)amid;
3-(2-Oxoimidazol-1-ylmethyl)-2-phenylchinolin-4-carbonsäure((S)-1-cyclohexylethyl)amid;
3-[3-Oxo-4-(2-pyrrolidin-1-ylethyl)piperazin-1-ymethyl]-2-phenylchinolin-carbonsäure((S)-1-cyclohexyethyl)amid;
3-[4-(2-Diethylaminoethyl)-3-oxopiperazin-1-ylmethyl)-2-pbenylchinolin-4-carbonsäure((S)-1-cyclohexyllethyl)amid;
3-[4-(2-Dimethylaminoethyl)-3-oxopiperazin-1-yhmethyl]-2-phenylchinolin-4-carbonsäure((S)-1-cyclohexylethyl)amid;
3-[4-(2-Aminoethyl)-3-oxopiperazin-1-ylmethyl]-2-phenylchinolin-4-carbonsäure((S)-1-cyclohexylethyl)amid,
3-[4-(2-Morpholin-4-ylethyl)-3-oxopiperazin-1-ylmethyl]-2-phenylchinolin-4-carbonsäure((S)-1-cyclohexylethyl)amid;
3-(4-Ethylcarbamoylpiperazin-1-ylmethyl)-2-phenylchinolin-4-carbonsäure((S)-1-cyclohexylethyl)amid;
3-(4-Isopropylcarbamoylpiperazin-1-ylmethyl)-2-phenylchinolin-4-carbonsäure((S)-1-cyclohexylethyl)amid;
3-(3-Oxopiperazin-1-ylmethyl)-2-thiophen-3-ylchinolin-4-carbonsäure((S)-1-cyclohexylethyl)amid; und
3-[4-(3-Dimethylaminopropyl)-3-oxopiperazin-1-ylmethyl]-6-fluor-2-phenylchinolin-4-carbonsäure((S)-1-cyclohexylethyl)amid; oder ein pharmazeutisch verträgliches Salz oder Solvat davon:

2. Arzneimittel, umfassend eine Verbindung gemäß Anspruch 1 und einen pharmazeutisch verträglichen Träger.

3. Verbindung gemäß Anspruch 1 zur Verwendung als therapeutischer Wirkstoff.

4. Verbindung gemäß Anspruch 1 für die Behandlung oder Vorbeugung von primären und sekundären Erkrankungen der Erfindung.

5. Verwendung einer Verbindung gemäß Anspruch 1 bei der Herstellung eines Medikaments für die Behandlung der primären und sekundären Erkrankungen der Erfindung.

## Revendications

1. Antagoniste des récepteurs de tachykinine, choisi dans le groupe consistant en :
((S)-1-cyclohexyl-éthyl)-amide d'acide 3-{[3-diéthylamino-propyl)-méthyl-amino]-méthyl}-2-phényl-quinoléine-4-carboxylique ;
((S)-1-cyclohexyl-éthyl)-amide d'acide 3-[1,4]-diazépan-1-ylméthyl-2-phényl-quinoléine-4-carboxylique ;
((S)-1-cyclohexyl-éthyl)-amide d'acide 3-dipropylaminométhyl-2-phényl-quinoléine-4-carboxylique ;
((S)-1-cyclohexyl-éthyl)-amide d'acide 3-[(1-benzyl-pipéridine-4-ylamino)-méthyll-2-phényl-quinoléine-4-carboxylique ;
((S)-1-cyclohexyl-éthyl)-amide d'acide 3-(indan-2-ylaminométhyl)-2-phényl-quinoléine-4-carboxylique ;
((S)-1-cyclohexyl-éthyl)-amide d'acide 2-phényl-3-thiazolidine-3-ylméthyl-quinoléine-4-carboxylique ;
((S)-1-cyclohexyl-éthyl)-amide d'acide 3-{[benzyl-(2-hydroxy-éthyl)-amino]-méthyl}-2-phényl-quinoléine-4-carboxylique ;
((S)-1-cyclohexyl-éthyl)-amide d'acide 3-(2,3-dihydro-indol-1-ylméthyl)-2-phényl-quinoléine-4-carboxylique ;
((S)-1-cyclohexyl-éthyl)-amide d'acide 3-butylaminométhyl-2-phényl-quinoléine-4-carboxylique ;
((S)-1-cyclohexyl-éthyl)-amide d'acide 3-((R)-3-hydroxy-pyrrolidine-1-ylméthyl)-2-phényl-quinoléine-4-carboxylique ;
((S)-1-cyclohexyl-éthyl)-amide d'acide 3-diméthylaminométhyl-2-phényl-quinoléine-4-carboxylique ;
((S)-1-cyclohexyl-éthyl)-amide d'acide 3-((S)-hydroxy-pyrrolidine-1-ylméthyl)-2-phényl-quinoléine-4-carboxylique ;
((S)-1-cyclohexyl-éthyl)-amide d'acide 3-(3,4-dihydro-1H-isoquinoléine-2-ylméthyl)-2-phényl-quinoléine-4-carboxylique ;
((S)-1-cyclohexyl-éthyl)-amide d'acide 3-{[méthyl-(2,2,6,6-tétraméthyl-pipéridine-4-yl)-amino]-méthyl}-2-phényl-quinoléine-4-carboxylique ;
((S)-1-cyclohexyl-éthyl)-amide d'acide 3-(4-oxo-pipéridine-1-ylméthyl)-2-phényl-quinoléine-4-carboxylique ;
((S)-1-cyclohexyl-éthyl)-amide d'acide 6-fluoro-3-[3-oxo-4-(2-pipéridine-1-yl-éthyl)-pipérazine-1-ylméthyl]-2-phényl-quinoléine-4-carboxylique ;
((S)-1-cyclohexyl-éthyl)-amide d'acide 3-(3,4-dihydro-1H-pyrrolo-[1,2-a]pyrazine-2-ylméthyl)-2-phényl-quinoléine-4-carboxylique ;
((S)-1-cyclohexyl-éthyl)-amide d'acide 3-[3-oxo-4-(3-pipéridine-1-yl-propyl)-pipérazine-1-ylméthyl]-2-phényl-quinoléine-4-carboxylique ;
((S)-1-cyclohexyl-éthyl)-amide d'acide 3-(1-oxo-3,4-dihydro-1H-pyrrolo[1,2-a]pyrazine-2-ylméthyl)-2-phényl-quinoléine-4-carboxylique ;
((S)-1-cyclohexyl-éthyl)-amide d'acide 3-diméthylaminométhyl-6-fluoro-2-phényl-quinoléine-4-carboxylique ;
((S)-1-cyclohexyl-éthyl)-amide d'acide 6-fluoro-3-(1-oxo-3,4-dihydro-1H-pyrrolo[1,2-a]pyrazine-2-ylméthyl)-2-phényl-quinoléine-4-carboxylique ;
((S)-1-cyclohexyl-éthyl)-amide d'acide 3-(4-oxo-1-phényl-1,3,8-triaza-spiro[4.5]dec-8-ylméthyl)-2-phényl-quinoléine-4-carboxylique ;
((S)-1-cyclohexyl-éthyl)-amide d'acide 3-[4-(3-diméthylaminopropyl)-3-oxo-pipérazine-1-ylméthyl]-2-phényl-quinoléine-4-carboxylique ;
((S)-1-cyclohexyl-éthyl)-amide d'acide ditrifluoroacétate de 3-(4-méthyl-3-oxo-pipérazine-1-ylméthyl)-2-phényl-quinoléine-4-carboxylique ;
((S)-1-cyclohexyl-éthyl)-amide d'acide 3-(4-éthyl-3-oxo-pipérazine-1-ylméthyl)-2-phényl-quinoléine-4-carboxylique ; ((S)-1-cyclohexyl-éthyl)-amide d'acide 3-(2-oxo-imidazolidine-1-ylméthyl)-2-phényl-quinoléine-4-carboxylique ;
((S)-1-cyclohexyl-éthyl)-amide d'acide 3-[3-oxo-4-(2-pyrrolidine-1-yl-éthyl)-pipérazine-1-ylméthyl]-2-phényl-quinoléine-4-carboxylique ;
((S)-1-cyclohexyl-éthyl)-amide d'acide 3-[4-(2-diéthylamino-éthyl)-3-oxo-pipérazine-1-ylméthyl]-2-phényl-quinoléine-4-carboxylique ;
((S)-1-cyclohexyl-éthyl)-amide d'acide 3-[4-(2-diméthylamino-éthyl)-3-oxo-pipérazine-1-ylméthyl]-2-phényl-quinoléine-4-carboxylique ;
((S)-1-cyclohexyl-éthyl)-amide d'acide 3-[4-(2-amino-éthyl)-3-oxo-pipérazine-1-ylméthyl]-2-phényl-quinoléine-4-carboxylique ;
((S)-2-cyclohexyl-éthyl)-amide d'acide 3-[4-(2-morpholine-4-yl-éthyl)-3-oxo-pipérazine-1-ylméthyl]-2-phényl-quinoléine-4-carboxylique ;
((S)-1-cyclohexyl-éthyl)-amide d'acide 3-(4-éthylcarbamoyl-pipérazine-1-ylméthyl)-2-phényl-quinoléine-4-carboxylique ;
((S)-1-cyclohexyl-éthyl)-amide d'acide 3-(4-isopropylcarbamoyl-pipérazine-1-ylméthyl)-2-phényl-quinoléine-4-carboxylique ;
((S)-1-cyclohexyl-éthyl)-amide d'acide 3-(3-oxo-pipérazine-1-ylméthyl)-2-thiophène-3-yl-quinoléine-4-carboxylique ;
((S)-1-cyclohexyl-éthyl)-amide d'acide 3-[4-(3-diméthylaminopropyl)-3-oxo-pipérazine-1-ylméthyl]-6-fluoro-2-phényl-quinoléine-4-carboxylique ;
ou un de ses sels ou produits de solvatation pharmaceutiquement acceptables.

2. Composition pharmaceutique comprenant un composé suivant la revendication 1 et un support pharmaceutiquement acceptable.

3. Composé suivant la revendication 1, destiné à être utilisé comme substance thérapeutique active.

4. Composé suivant la revendication 1, pour le traitement ou la prophylaxie des Affections Primaires et Affections Secondaires de la présente invention.

5. Utilisation d'un composé suivant la revendication 1, dans la production d'un médicament destiné au traitement des Affections Primaires et Affections Secondaires de la présente invention.
